# EUROPEAN PATENT APPLICATION

(11) **EP 4 012 413 A1**
(43) Date of publication of application: **15.06.2022**
(21) Application number: 20212397.2
(22) Date of filing: 08.12.2020
(51) Int. Cl.: G01N 33/574

(54) **HGF AS A MARKER FOR PRGRESSION OF OVARIAN CANCER**

(71) Applicant: Technische Universität Dresden, 01069 Dresden (DE)
(72) Inventor: Klotz, Daniel Martin, 01277 Dresden (DE)
(74) Representative: Hertin und Partner Rechts- und Patentanwälte PartG mbB

(57) **Abstract**

The invention relates to a method for prognosing disease progression in a patient that has been diagnosed with ovarian cancer, wherein the method comprises determining a level of hepatocyte growth factor (HGF) in a sample from the patient, wherein said level of HGF in said sample is indicative of a subsequent adverse event in the health of said patient. In embodiments, the adverse event is cancer progression or recurrence after therapeutic intervention, such as after surgical removal and/or chemotherapy; detection and/or development of chemotherapy resistance; and/or death. The invention also relates to a kit for carrying out the method of the invention.

## Description

The invention relates to a method for prognosing disease progression in a patient that has been diagnosed with a ovarian cancer, wherein the method comprises determining a level of hepatocyte growth factor (HGF) in a sample from the patient, wherein said level of HGF in said sample is indicative of a subsequent adverse event in the health of said patient. In embodiments, the adverse event is cancer progression or recurrence after therapeutic intervention, such as after surgical removal and/or chemotherapy; detection and/or development of chemotherapy resistance; and/or death. The invention also relates to a kit for carrying out the method of the invention.

### BACKGROUND OF THE INVENTION

The pleiotropic protein hepatocyte growth factor (HGF) was originally discovered in 1984 as a mitogen for primary hepatocytes, which promotes motility of epithelial cells (9). So far, HGF is the only known ligand of the receptor tyrosine kinase mesenchymal-epithelial transition (cMET), which is encoded by the MET proto-oncogene (10,11). HGF is synthesized in an inactive single-chain form, referred to as pro-HGF, which can be activated by proteolytic cleavage (12). The canonical activation of the HGF/cMET signaling axis is initiated by the binding of HGF to cMET on the cellular surface, inducing cMET homodimerization and autophosphorylation, followed by the activation of multiple adapter proteins and downstream pathways, such as the mitogen-activated protein kinases (MAPK), the phosphoinositide 3-kinases (PI3K), or the signal transducer and activator of transcription 3 (STAT3) pathway (13,14). HGF-signaling is functionally involved into the development of the ovary and the follicles in terms of paracrine signaling between HGF and cMET (15,16). In the adult ovary, HGF is produced by ovarian mesenchymal stroma and theca cells, whereas the ovarian surface epithelium and granulosa cells express only cMET (17). This may provide an explanation of the link of this signaling pathway in malignant ovarian transformation.

The HGF/cMET pathway controls a variety of cellular functions, such as proliferation, angiogenesis or migration and has also been associated with the metastatic progression of several human cancer (18-21). In ovarian cancer, the HGF/cMET pathway is aberrantly activated and overexpression of cMET has been observed along a wide range of incidence (10-75%) across all histologic subtypes (22) and was mostly associated with poor prognosis (23,24). Moreover, there is evidence from in vitro studies, that HGF-signaling contributes to matrix metalloprotease 9 (MMP9) mediated invasion and migration of ovarian cancer cells (25). Therefore, HGF/cMet signaling has been tested as a therapeutic target for ovarian cancer (26,27). The actual clinical relevance of circulating HGF for ovarian cancer patients, especially in the course of the disease, is unknown.

Ovarian cancer is the leading cause of death among females with gynecological malignancies and more than 70% of patients are diagnosed with advanced disease (1). Standard therapy of advanced ovarian cancer comprises surgical debulking and chemotherapy. The first aims at complete macroscopic tumor resection, the latter consists of platinum- and paclitaxel-based chemotherapy and maintenance treatment, all of which improves survival (2-4). The most important clinical prognostic factor is the postoperative residual tumor burden in advanced ovarian cancer (1,5). However, despite improved radical surgical debulking and the implementation of innovative targeted maintenance therapies to standard treatment, such as anti-angiogenetic therapy with bevacizumab or PARP-inhibitors for selected patients, the majority of patients with advanced ovarian cancers still face a poor overall prognosis (6-8).

In light of the prior art, there remains a significant need in the art for the discovery of blood-based predictive and/or prognostic biomarkers for ovarian cancer is of high clinical interest. Accordingly, the problem underlying the present invention is the provision of a method for the prognosis of ovarian cancer, based on a suitable prognostic biomarker.

### SUMMARY OF THE INVENTION

In light of the prior art the technical problem underlying the present invention is to provide alternative and/or improved methods for the biomarker-based prognosis of diagnosed ovarian cancer.

This problem is solved by the features of the independent claims. Preferred embodiments of the present invention are provided by the dependent claims.

The invention therefore relates to a method for prognosing disease progression in a patient that has been diagnosed with ovarian cancer, wherein the method comprises determining a level of hepatocyte growth factor (HGF) in a sample from the patient, wherein said level of HGF in said sample is indicative of a subsequent adverse event in the health of said patient.

The present invention is based on the unexpected finding that the level HGF in a sample from a patient that has been diagnosed with ovarian cancer can serve as an independent prognostic biomarker for disease progression and preferably therapy guidance. Importantly, the sample may be isolated at the time point of diagnosis and/or at a later stage after diagnosis. For example, the sample can be isolated at a time point during an ongoing treatment that has been initiated after diagnosis, such as after surgical removal of the tumor, before initiation of a chemotherapy, during an ongoing chemotherapy, and/or after competition of a chemotherapy. The HGF level can therefore be indicative of the effectiveness of an ongoing or initiated therapy.

It is understood that in the context of the present invention the "time point of diagnosis" is the time point at which the treating physician or medical personnel suspects the patient of having ovarian cancer. At this time point, the treating personnel decides on the next steps for confirming the diagnosis (surgery/biopsy isolation and histological analysis, therapeutic measures, etc.). This is the earliest time point of sample isolation in the context of the method of the present invention and corresponds to the time point, from which the patient is classified as "diagnosed" with ovarian cancer, although a histological proof of the ("suspected and later on confirmed") diagnosis may not be available, yet. However, the sample or samples to be used in the context of the present invention may have been isolated at any one or more time points after "the time point of diagnosis", such as a time point before surgery or after primary surgery, or a time point before, during or after chemotherapy.

In embodiments, the sample has been isolated from the patient not more than about 1 month before histological confirmation of the diagnosis, such as 4 weeks, 3 weeks, 2 weeks, or 1 week, 6 days, 5 days, 4 days, 3 days, 2 days, 1 day before histological confirmation of the diagnosis.

In embodiments, a treatment can comprise surgical removal, radiation therapy and/or chemotherapy. In embodiments, an initial or routine treatment initiated after primary diagnosis of the cancer can comprise surgical removal of the tumor (primary surgery) and adjuvant chemotherapy, which is administered after surgery. In embodiments, the level of HGF can be indicative of the effectiveness of a chemotherapy and/or can be indicative of chemotherapy resistance of the tumor/cancer of the patient.

In the context of the present invention, the terms "tumor" and "cancer" are used interchangeably.

It was surprisingly found that high levels of HGF in a sample isolated from the patient at the time point of diagnosis or thereafter, for example during an ongoing therapy that has been initiated after diagnosis of the cancer, is indicative of the occurrence of an adverse event. In the context of the invention, such an adverse event may be the recurrence of a tumor after primary surgery, occurrence of chemotherapy resistance of a tumor/of residual cancer cells remaining in the patient after primary surgery and/or radiation therapy, or death of the patient.

In embodiments, the method of the invention comprises additionally risk stratification, therapy guidance, therapy stratification and/or therapy monitoring of the patient.

The method of the present invention enables risk stratification of the patient based on the HGF level in the sample. For example, a patient with a HGF level that above a certain threshold level, which may be specific for the time point of sample isolation and/or of the kind of adverse event, can be categorized as a high risk patient with respect to the respective adverse event. In embodiments, a patient with a comparably high HGF level after initiation of an adjuvant chemotherapy, for example after 1, 2, or 3 cycles of an adjuvant chemotherapy of a total of 4 cycles, may be indicative of resistance of the tumor cells to the chemotherapy. Accordingly, the respective patient can be categorized as a high-risk patient for the development or presence of chemotherapy resistance. Furthermore, such a result can also be indicative for a requirement to modify an ongoing (primary/routine/standard) therapy, or, if the sample has been isolated from the patient upon diagnosis and before initiation of primary therapy, the method can be used to select a different (non-routine) primary therapy based on the determined HGF level. Therefore, the method of the invention can also be used for therapy monitoring, therapy guidance and therapy stratification. Importantly, since in embodiments HGF can be indicative of the occurrence of the adverse event even before initiation of therapy or of certain therapeutic measures, the method can be used for determining a personalized therapeutic approach for the patient before initiation of (primary) therapy, in particular chemotherapy.

In embodiments, the ovarian cancer of the present invention is associated with BRCA1 and/or BRCA2 mutations. In embodiments, the ovarian cancer is wild type with respect to BRCA1 and/or BRCA2.

In embodiments, the method of the present invention comprises all kind of patients diagnosed with ovarian cancer, irrespective of whether the cancer actually displays BRCA1 and/or BRCA2 mutations, whether the cancer cells carry wild type (wt) alleles of BRCA1 and/or BRCA2, or whether the patient carries a germline mutation in BRCA1 and/or BRCA2.

Importantly, certain therapeutic measures are currently only performed on newly diagnosed ovarian cancer patients with BRCA1 and/or BRCA2 mutations, while cancer patients with ovarian cancer but without BRCA1 and/or BRCA2 mutations do not typically receive such measures at present. For example, PARP inhibitors have been shown to be effective in patients with advanced BRCA1/2 mutant ovarian cancer and are used in first-line therapy of such patients, while patients with wt BRCA1/2 alleles do not receive PARP inhibitors in combination with bevacizumab. Importantly, the method of the present invention can be used to identify high-risk cancer patients with wt BRCA1/2 alleles that may benefit from administration of PARP inhibitors in the context of primary therapeutic measures and can therefore be used for therapy guidance and patient stratification.

In embodiments of the invention, the method comprises determining additional clinicopathological parameters, such as one or more biomarkers and/or one or more clinical scores, in particular FIGO staging and/or determining a level of CA125 in a sample from the patient. Combinatorial analysis of HGF with additional clinicopathological parameters can offer important additional information.

In embodiments, the subsequent adverse event in the health of said patient is cancer progression after therapeutic intervention; cancer recurrence after surgical removal and/or chemotherapy, such as cancer recurrence within 6 months after completion of chemotherapy; detection and/or development of chemotherapy resistance; and/or death.

In embodiments, the method is used for determining the likelihood of disease progression or recurrence after diagnosis and performance of a treatment regimen, such as surgery in combination with adjuvant chemotherapy aimed at eradicating the cancer. In that sense, the method can be used for prognosing the time of progression free survival of the patient, for example it can be used for determining the likelihood of recurrence of the cancer within 2, 3, 4, 5, 6, 7, 8, 9, 10, 11, 12, 13, 14, 15, 16, 17, 18, 20, 22, or 24, 36, 48 and 60 months from the time point of diagnosis or preferably from the time point of finishing the respective (routine/standard) treatment regimen.

In further embodiments, the method of the invention can be used for prognosing the time of overall survival of the patient. The method can be used for prognosing the death of a patient within a certain time interval, such as within 2, 3, 4, 5, 6, 7, 8, 9, 10, 11, 12, 13, 14, 15, 16, 17, 18, 20, 22, or 24, 36, 48, 60 months from the time point of diagnosis or preferably from the time point of finishing the respective (routine/standard) treatment regimen.

In further embodiments, the method of the invention can be used for prognosing the detection and/or development of chemotherapy resistance, such as in particular platinum resistance, after primary diagnosis of the cancer. The method can be used for prognosing the detection and/or development of chemotherapy resistance of a patient within a certain time interval, such as within 1, 2, 3, 4, 5 or 6 months from initiation of primary chemotherapy, such as platinum chemotherapy resistance and/or PARP inhibitor resistance. In embodiments, the method of the invention can be used for prognosing the detection and/or development of (platinum) chemotherapy resistance or therapy resistance (such as but not restricted to resistance to administration of PARP inhibitor, anti-angiogenesis therapy or immunotherapy) of a patient within a certain time interval, such as within 1, 2, 3, 4, 5, 6, 8, 10, 12, 15, 18, 21, 24 months from initiation of primary chemotherapy. The method of the invention can therefore in embodiments address the clinical need to predict whether or not a patient will be resistant to a chemotherapy, such as a standard platinum-based chemotherapy, which can be indicated by a disease relapse within the first 6 months after completion of chemotherapy. Resistance to chemotherapeutic agents, such as platinum resistance, is indicative of poor prognosis and the method of the invention enables identifying those patients prior to treatment. This allows to select a personalized and potentially more targeted therapeutic strategy that takes into account the prognosed development of chemotherapy resistance. This would also avoid the side effects seen by platinum- and paclitaxel-based first line therapy. Measuring HGF levels prior to treatment offers such a prognostic test with respect to cancer recurrence after standard therapy and development/detection of chemotherapy resistance.

In embodiments, the method of the invention can also be used for prognosing and/or prediction of resistance of a patient to platinum chemotherapy and/or PARP inhibitor therapy. Importantly, in certain clinical setting/according to certain guidelines PARP inhibitors can only be given if to patients responding to platinum chemotherapy. Furthermore, a resistance to subsequent platinum therapy may correlate with PARP inhibitor resistance during maintenance treatment. It has been reported that secondary platinum resistance may correlate with a shorter time of PARP inhibitor maintenance treatment.

After diagnosis of ovarian cancer, an initial treatment regimen (primary treatment) will be performed on the patient, which is herein referred to as a therapeutic intervention after diagnosis. Such a primary treatment/primary therapeutic invention can be composed of one or more therapeutic measures selected from the group of surgical removal of the cancer/tumor mass, radiation therapy, chemotherapy, chemotherapy using platin- and/or taxan-based chemotherapy agents, administration of non-routine therapeutic agents, such as PARP inhibitors, Bevacizumab and/or modulators of HGF signaling, such as cMet inhibitors.

As used herein, a routine primary treatment of an ovarian cancer refers to the standard therapy that is usually administered to a cancer patient depending on the type of cancer and the stage of cancer progression. For example, in case of a diagnosed ovarian cancer stage I-IV (FIGO staging), routine treatment includes primary radical surgery aiming at macroscopic complete tumor resection followed by adjuvant platinum- and taxane-adjuvant chemotherapy. In case of no contraindication, patients with at least a FIGA stage IIIb may routinely additionally be treated with the monoclonal antibody bevacizumab. In case of BRCA-mutant and/or homologous recombination deficient (HRD) ovarian cancer, these patients will also receive olaparib as maintenance treatment. In case of an absence of HRD, patients can also receive niraparib instead of bevacizumab in the maintenance therapy.

Based on the result of the method of the present invention, a routine treatment of the diagnosed ovarian cancer patient can be modified, if the determined HGF level is indicative of a subsequent adverse event in case of initiation or continuation of the routine treatment, depending on the time point of sample isolation from the patient (i.e., before, during or after performance of a routine treatment).

In embodiments of the method of the present invention, the patient will receive a primary treatment that includes surgical removal of the cancer/tumor mass (primary surgery). In embodiments, primary treatment can include radiation therapy, also in combination with primary surgery. In embodiments, primary treatment can include chemotherapy therapy, also in combination with primary surgery and/or radiation therapy.

Surgical removal will in many cases be combined with chemotherapy and potentially also with radiation therapy.

In embodiments, the therapeutic intervention and/or chemotherapy include administration of platin- and/or taxan-based chemotherapy agents.

In embodiments, the sample from the patient is a blood sample, such as a serum, plasma or whole blood sample.

In embodiments of the method of the invention, the sample has been isolated upon initial diagnosis of said cancer and before initiation of therapeutic measures.

In embodiments, the sample has been isolated after therapeutic intervention, for example after surgical removal of affected tissues.

In the context of the invention, the sample may have been isolated before, during or after therapeutic intervention, such as before, during or after administration of chemotherapy.

It is a surprising advantage of the present invention that the level of HGF is indicative of the occurrence of adverse events throughout the course of the diagnosed disease. For example, HGF can be determined directly after diagnosis and before initiation of therapy, but can also be determined after (primary) therapy has been initiated and HGF levels can be used to monitor success of an ongoing therapy or to observe disease progression after completion of a therapy regimen. Accordingly, the present HGF based method can be used to determine an initial recommended treatment regimen, and/or to modify an ongoing treatment regimen, and/or to decide on initiating a further treatment regimen after a previous treatment regimen has been finished. Importantly, the method of the invention can involve determining HGF in two or more samples that have been isolated from the patient at several time points at or after diagnosis.

In embodiments, the method of the invention comprises determining the level of HGF in two more samples from the patient isolated at the time point of diagnosis and/or thereafter. In such embodiments, the patient specific dynamic of HGF levels after diagnosis of the cancer can be an independent predicator of an adverse event, such as death of the patient. In such embodiments, the dynamic of the determined HGF levels over the course of disease can be used for the prognosis of an adverse event in the health of the patient, such as cancer recurrence or death.

In embodiments, the method of the invention comprises determining a HGF level in a sample from the patient at more than one time points at and/or after diagnosis of said cancer. In other words, HGF is measured in samples that have been isolated at different time points, such as in a sample that has been isolated at the time point of diagnosis and in a sample that has been isolated at a later time point after diagnosis. Also a first sample for determining HGF could be isolated at an earlier time point after diagnosis and a second sample could be isolated at a second later time point. Additional samples could also be provided.

In embodiments, the method comprises:
- providing a sample from said patient, and
- determining a level of HGF in said sample,
- wherein said level of HGF indicates a likelihood of an adverse event in the health of said patient.

In embodiments of the method of the invention, a determined level of HGF equal to or below a threshold value (low severity level) is indicative of the absence of a subsequent adverse event in the health of said patient, and a determined level of HGF above said threshold value (high severity level) is indicative of the occurrence of a subsequent adverse event in the health of said patient.

In embodiments, the patient has been diagnosed with ovarian cancer, the sample has been isolated at the time point of primary diagnosis and the adverse event is cancer recurrence, preferably within 6 months from the termination of a primary treatment regimen, and/or death of the patient.

In embodiments, the patient has been diagnosed with ovarian cancer, the sample has been isolated before primary surgery. In embodiments, the patient has been diagnosed with ovarian cancer, the sample has been isolated after primary surgery and before initiation of adjuvant chemotherapy, during adjuvant chemotherapy or after completion of adjuvant chemotherapy, wherein the adverse event may be cancer recurrence, preferably within 6 months from the termination of a primary treatment regimen, and/or death of the patient, preferably within 6 months from the termination of a primary treatment regimen.

In embodiments of the invention, an HGF level above a certain threshold level is an independent predictor of the occurrence of an adverse event such as cancer recurrence or death after completion of a primary routine/standard therapeutic regimen.

The HGF threshold levels disclosed herein preferably relate to HGF levels determined in a blood sample, such as preferably a serum sample.

In embodiments, the high severity level of HGF or fragment(s) thereof is above 400 pg/ml, preferably 401 pg/ml. In embodiments, the sample has been isolated at primary diagnosis, the adverse event is cancer progression or recurrence after therapy and the high severity level of HGF or fragment(s) thereof is above 400 pg/ml, preferably 401 pg/ml.

In embodiments, the high severity level of HGF or fragment(s) thereof is above 900 pg/ml, preferably 970 pg/ml. In embodiments, the sample has been isolated at primary diagnosis, the adverse event is death and the high severity level of HGF or fragment(s) thereof is above 900 pg/ml, preferably 970 pg/ml.

In embodiments, the high severity level of HGF or fragment(s) thereof is above 1500 pg/ml, preferably 1516 pg/ml. In embodiments, the sample has been isolated after primary surgery, preferably within 1 week after primary surgery, the adverse event is cancer progression or recurrence after therapy and the high severity level of HGF or fragment(s) thereof is above 1500 pg/ml, preferably 1516 pg/ml.

In embodiments, the high severity level of HGF or fragment(s) thereof is above 2100 pg/ml, preferably 2122 pg/ml. In embodiments, the sample has been isolated after primary surgery, preferably within 1 week after primary surgery, the adverse event is death and the high severity level of HGF or fragment(s) thereof is above 2100 pg/ml, preferably 2122 pg/ml.

In embodiments, the high severity level of HGF or fragment(s) thereof is above 800 pg/ml, preferably 826 pg/ml. In embodiments, the sample has been isolated before chemotherapy, preferably more than 1 week after primary surgery but before chemotherapy, the adverse event is cancer progression or recurrence after therapy and the high severity level of HGF or fragment(s) thereof is above 800 pg/ml, preferably 826 pg/ml.

In embodiments, the high severity level of HGF or fragment(s) thereof is above 850 pg/ml, preferably 860 pg/ml. In embodiments, the sample has been isolated before chemotherapy, preferably more than 1 week after primary surgery but before chemotherapy, the adverse event is death and the high severity level of HGF or fragment(s) thereof is above 850 pg/ml, preferably 860 pg/ml.

In embodiments, the high severity level of HGF or fragment(s) thereof is above 480 pg/ml, preferably 487 pg/ml. In embodiments, the sample has been isolated after three cycles of chemotherapy, the adverse event is cancer progression or recurrence after therapy and the high severity level of HGF or fragment(s) thereof is above 480 pg/ml, preferably 487 pg/ml.

In embodiments, the high severity level of HGF or fragment(s) thereof is above 530 pg/ml, preferably 537 pg/ml. In embodiments, the sample has been isolated after three cycles of chemotherapy, the adverse event is death and the high severity level of HGF or fragment(s) thereof is above 530 pg/ml, preferably 537 pg/ml.

In embodiments, the high severity level of HGF or fragment(s) thereof is above 600 pg/ml, preferably 603 pg/ml. In embodiments, the sample has been isolated after completion of chemotherapy, the adverse event is cancer progression or recurrence after therapy and the high severity level of HGF or fragment(s) thereof is above 600 pg/ml, preferably 603 pg/ml.

In embodiments, the high severity level of HGF or fragment(s) thereof is above 480 pg/ml, preferably 489 pg/ml. In embodiments, the sample has been isolated after completion of chemotherapy, the adverse event is death and the high severity level of HGF or fragment(s) thereof is above 480 pg/ml, preferably 489 pg/ml.

In embodiments, the high severity level of the dynamic range of HGF or fragment(s) thereof is above 4260 pg/ml, preferably 4261 or 4266 pg/ml, wherein preferably the adverse event is cancer progression or recurrence after therapy or death, respectively. The dynamic range of HGF is measured across primary treatment (determined as the area under the curve of two or more individual time points). Accordingly, in such embodiments at least two (or more) samples isolated at different time points are analyzed for the level of HGF. Samples can be isolated throughout primary treatment, preferably as stated before.

In embodiments, the high severity level of HGF or fragment(s) thereof is above 340 pg/ml, preferably 347 pg/ml. In embodiments, the ovarian cancer is BRCA1 and/or BRCA2 positive (carries a germline or somatic mutation of BRCA1 and/or BRCA2), the adverse event is cancer progression or recurrence after therapy and the high severity level of HGF or fragment(s) thereof is above 340 pg/ml, preferably 347 pg/ml. Genetic confirmation of (germ line or somatic) BRCA1 mutations, BRCA2 mutations or wild type BRCA1/2 status can be obtained before or after samples have been taken for determining a HGF level in a patient that has been diagnosed with ovarian cancer. Samples can be obtained before and throughout primary treatment, preferably at primary diagnosis, as disclosed herein.

In embodiments, the high severity level of HGF or fragment(s) thereof is above 950 pg/ml, preferably 954 pg/ml. In embodiments, the ovarian cancer is BRCA1 and/or BRCA2 positive (carries a germline or somatic mutation), the adverse event is death and the high severity level of HGF or fragment(s) thereof is above 950 pg/ml, preferably 954 pg/ml. Genetic confirmation of (germ line or somatic) BRCA1 mutations, BRCA2 mutations or wild type BRCA1/2 status can be obtained before or after samples have been taken for determining a HGF level in a patient that has been diagnosed with ovarian cancer. Samples can be obtained before and throughout primary treatment, preferably at primary diagnosis, as disclosed herein.

In embodiments, the high severity level of HGF or fragment(s) thereof is above 710 pg/ml, preferably 716 pg/ml. In embodiments, the ovarian cancer is BRCA1 and/or BRCA2 wild type (carries no mutation), the adverse event is cancer progression or recurrence after therapy and the high severity level of HGF or fragment(s) thereof is above 710 pg/ml, preferably 716 pg/ml. Genetic confirmation of (germ line or somatic) BRCA1 mutations, BRCA2 mutations or wild type BRCA1/2 status can be obtained before or after samples have been taken for determining a HGF level in a patient that has been diagnosed with ovarian cancer. Samples can be obtained before and throughout primary treatment, preferably at primary diagnosis, as disclosed herein.

In embodiments, the high severity level of HGF or fragment(s) thereof is above 720 pg/ml, preferably 728 pg/ml. In embodiments, the ovarian cancer is BRCA1 and/or BRCA2 wilt type (carries no mutation), the adverse event is death and the high severity level of HGF or fragment(s) thereof is above 720 pg/ml, preferably 728 pg/ml. Genetic confirmation of (germ line or somatic) BRCA1 mutations, BRCA2 mutations or wild type BRCA1/2 status can be obtained before or after samples have been taken for determining a HGF level in a patient that has been diagnosed with ovarian cancer. Samples can be obtained before and throughout primary treatment, preferably at primary diagnosis, as disclosed herein.

In embodiments, the high severity level of HGF or fragment(s) thereof is above 1500 pg/ml, preferably 1550 pg/ml. In embodiments, the adverse event is therapy resistance, such as preferably chemotherapy resistance and in particular platinum resistance, and the high severity level of HGF or fragment(s) thereof is above 1500 pg/ml, preferably 1550 pg/ml. In such embodiments, the (one or more) sample can be isolated at various time points, for example upon initial diagnosis, before and/or across primary treatment (e.g., initial diagnosis, after surgery, before chemotherapy), but preferably at/upon initial diagnosis of ovarian cancer, after surgery (e.g., within one week after primary surgery) and/or before chemotherapy (e.g. more than one week after primary surgery and before onset of chemotherapy). This cut off is irrespective of other biomarker status, such as but not restricted to BRCA1/2 mutational status.

In embodiments, the high severity level of HGF or fragment(s) thereof is above 450 pg/ml, preferably 456 pg/ml. In embodiments, the sample has been isolated during or after completion of chemotherapy, preferably after completion of first-line chemotherapy, the adverse event is therapy resistance, such as preferably chemotherapy resistance and in particular platinum resistance, and the high severity level of HGF or fragment(s) thereof is above 450 pg/ml, preferably 456 pg/ml. The use of this threshold level during or after primary (standard) chemotherapy allows a particularly advantageous assessment of ongoing treatment strategy and can be particularly helpful for deciding to switch to a different treatment regime, such as an experimental treatment option. This cut off is irrespective of other biomarker status, such as but not restricted to BRCA1/2 mutational status.

As used herein, a time point of sample isolation after surgery preferably relates to a time point within one week after (primary) surgery, but before onset of subsequent chemotherapy. In preferred embodiments, the patient underwent surgery and subsequent chemotherapy, wherein onset of chemotherapy is more than one week after surgery. Furthermore, when referring to a time point of sample before chemotherapy, this time point is preferably more than one week after a potential surgery for removing the tumor and before onset of a (subsequent) chemotherapy.

In embodiments of the invention,
- the low severity level of HGF indicates initiation or continuation of a routine treatment of said cancer, or
- the high severity level of HGF indicates modifying a routine treatment of said cancer.

In preferred embodiments, modifying the treatment involves a change in one or more of the chemotherapeutic agents, inclusion of additional therapeutic agents in the treatment (and maintenance treatment), such as PARP inhibitors, Bevacizumab and/or modulators of HGF signaling.

Furthermore, in one aspect the present invention relates to a kit for carrying out the method for prognosing disease progression in a patient that has been diagnosed with ovarian cancer according to the method of the invention, comprising:
- detection reagents for determining the level of HGF in a sample from a patient, and
- reference data, or means to obtain reference data, for the risk of a patient as to whether a subsequent adverse event in the health of said patient will occur, in particular reference data corresponding to HGF severity levels disclosed herein and/or corresponding to a risk threshold or cut-off value, wherein preferably said reference data is stored on a computer readable medium and/or employed in the form of computer executable code configured for comparing the determined levels of HGF with the reference data, such as the HGF severity levels, risk threshold or cut-off value.

In the context of the present disclosure it is understood that features that are disclosed in the context of embodiments of the method of the present invention also relate to the kit of the invention and are therefore also disclosed in the context of the kit of the invention, and the other way around. Accordingly, all mandatory and optional features of the method of the invention are herewith also disclosed in the context of the kit of the invention, and vice versa.

### DETAILED DESCRIPTION OF THE INVENTION

The present invention is directed to a method for prognosing disease progression in a patient that has been diagnosed with ovarian cancer, wherein the method comprises determining a level of hepatocyte growth factor (HGF) in a sample from the patient, wherein said level of HGF in said sample is indicative of a subsequent adverse event in the health of said patient.

As used herein, the "patient" or "subject" may be a vertebrate. In the context of the present invention, the term "subject" includes both humans and animals, particularly mammals, and other organisms. Preferably, the terms patient or subject refer to a human being.

The method of the invention can refer to a patient diagnosed with ovarian cancer displaying/carrying BRCA1 and/or BRCA2 mutations, or to a patient diagnosed with ovarian cancer not displaying or carrying BRCA1 and/or BRCA2 mutations (meaning that the cancer cells carry wt alleles of BRCA1 and/or BRCA2).

BRCA1 and BRCA2 are human genes that produce the corresponding tumor suppressor proteins termed BRCA1 and BRCA2. These proteins help repair damaged DNA and, therefore, play a role in ensuring the stability of each cell's genetic material. When either of these genes is mutated, or altered, such that its protein product is not made or does not function correctly, DNA damage may not be repaired properly. As a result, cells are more likely to develop additional genetic alterations that can lead to cancer. Specific inherited mutations in BRCA1 and BRCA2 most notably increase the risk of female breast and ovarian cancers, but they have also been associated with increased risks of several additional types of cancer, including prostate cancer, pancreas cancer and colon cancer. People who have inherited mutations in BRCA1 and BRCA2 tend to develop breast and ovarian cancers at younger ages than people who do not have these mutations.

In the context of the present invention, an "adverse event in the health of a patient" relates to events that indicate complications or worsening of the health state of the patient, including worsening quality of life. In the context of the present invention, the term adverse event this includes events that indicate ineffectiveness of an initial routine treatment of the patient diagnosed with ovarian cancer initiated after initial diagnosis. This in particular includes cancer progression or cancer recurrence after therapeutic intervention; cancer recurrence after surgical removal and/or chemotherapy, such as cancer recurrence within 6 months after completion of chemotherapy; detection and/or development of development of chemotherapy resistance; and death.

In embodiments of the invention, the adverse event is cancer progression or cancer recurrence after therapeutic intervention, such as cancer recurrence or progression within 1, 2, 3, 4, 5, 6, 7, 8, 9, 10, 11, 12, 13, 14, 15, 16, 17, 18, 19, 20, 21, 22, 23, 24, 36, 48, or 60 months after the initiation or last step of the primary therapeutic invention.

As used herein, the term cancer progression comprises embodiments where primary therapy resulted in a situation where the cancer/tumor could be removed from the patient to an extend that it was practically no longer detectable, for example due to complete macroscopic resection during surgery, optionally in combination with chemotherapy to also remove residual cells that may not be visible, but where the cancer reappeared after the primary therapy has been completed. Such embodiments, where the seemed to have disappeared (the patient appeared to be tumor-free) but reoccurs are also referred to as "cancer recurrence". However, "cancer progression" also relates to embodiments where a complete detectable disappearance of the cancer could not be achieved, but where for example the cancer could be reduced and further growth could be inhibited and/or controlled by primary treatment, and where the cancer cells start to grow and expand after some time.

Therapeutic intervention can comprise surgical intervention, including surgical removal of the cancer. Surgical removal of the cancer/tumor relates to an aimed complete macroscopic removal/resection of cancerous cells from the patient's body during surgery. In embodiment, a complete removal cannot be achieved due to the residual tumor cells that are not visible or detectable and therefore remain in the patient's body. Furthermore, in embodiments, the anatomical location of a tumor may prevent complete resection of the tumor mass. However, the term "surgical removal/resection of the cancer/tumor" comprises these embodiments, where a complete removal may not occur, either voluntarily or involuntarily.

In embodiments, the adverse event in the health of the patient can be or comprise the occurrence or detection of chemotherapy resistance of cancer cells. In embodiments, chemotherapy resistance may be detected or develop 1, 2, 3, 4, 5, 6,7 ,8, 9, 10, 11, 12, 13, 14, 15, 16, 17, 18, 19, 20, 21, 22, 23, 24 , 36, 48, or 60 months after the initiation or the last step of the therapeutic invention. In embodiments, chemotherapy resistance occurs during a primary chemotherapeutic treatment regimen.

In embodiments, therapy resistance, in particular chemotherapy resistance such as platinum resistance, refers to a disease relapse, such as cancer progression or recurrence after a defined time interval after a therapeutic intervention, such as 1, 2, 3, 4, 5, 6,7 ,8, 9, 10, 11, 12, 13, 14, 15, 16, 17, 18, 19, 20, 21, 22, 23, 24 , 36, 48, or 60 months after the initiation or the last step of the therapeutic invention, preferably about 6 months.

Chemotherapy resistance generally refers to cancers that do not respond to a certain chemotherapeutic agent. In the context of the invention, occurrence or detection of chemotherapy resistance comprises the development of a resistance over the course of a primary chemotherapy, or the presence of such a resistance from the beginning, which may however only become apparent after treatment has been initiated.

Chemotherapy resistance can develop over the course of a treatment, wherein a cancer that has been responding to a therapy suddenly begins to grow again. There are a few possible causes of resistance in cancer, one of which is the presence of small pumps on the surface of cancer cells that actively move chemotherapeutic agents from inside the cell to the outside. Cancer cells may produce high amounts of these pumps, known as p-glycoprotein, in order to protect themselves from chemotherapeutics. Medications to inhibit the function of p-glycoprotein are undergoing investigation and may be suitable second-line therapies. Another mechanism of resistance is gene amplification, a process in which multiple copies of a gene are produced by cancer cells. This overcomes the effect of drugs that reduce the expression of genes involved in replication. With more copies of the gene, the drug cannot prevent all expression of the gene and therefore the cell can restore its proliferative ability. Cancer cells can also cause defects in the cellular pathways of apoptosis (programmed cell death). As most chemotherapy drugs kill cancer cells in this manner, defective apoptosis allows survival of these cells, making them resistant. Many chemotherapy drugs also cause DNA damage, which can be repaired by enzymes in the cell that carry out DNA repair. Upregulation of these genes can overcome the DNA damage and prevent the induction of apoptosis. Mutations in genes that produce drug target proteins, such as tubulin, can occur which prevent the drugs from binding to the protein, leading to resistance to these types of drugs. Drugs used in chemotherapy can induce cell stress, which can kill a cancer cell; however, under certain conditions, cells stress can induce changes in gene expression that enables resistance to several types of drugs.

In preferred embodiments, the adverse event is cancer recurrence within 6 months after completion of chemotherapy, which preferably was applied as part of a primary treatment comprising surgical intervention and chemotherapy.

In further embodiments, the adverse event is death of the patient, such as death within 1, 2, 3, 4, 5, 6, 7 ,8, 9, 10, 11, 12, 13, 14, 15, 16, 17, 18, 19, 20, 21, 22, 23, 24 , 36, 48, or 60 months after the initiation or last step of the therapeutic invention.

In this context, it understood that a "therapeutic intervention" comprises the first intervention in reaction to an initial diagnosis, such as the administration of chemotherapy and/or surgical removal of the cancer during primary/initial treatment. However, in the context of the invention, an adverse event after therapeutic intervention can also relate to an adverse event occurring after a secondary or tertiary therapeutic intervention, such as after applying a second-line therapy.

Therapeutic interventions and treatment regimens that are applied in the context of the present invention, either as a routine treatment or as a modified treatment, can comprise all kinds of cancer therapy approaches. In the context of the present invention, this includes any kind of treatment of cancer, including, without limitation, surgery, chemotherapy, radiotherapy, irradiation therapy, hormonal therapy, targeted therapy, cellular therapy, cancer immunotherapy, monoclonal antibody therapy.

Administration of the compound can be individual as monotherapy or in combination with one or more other cancer therapies. In the context of the present invention the term "in combination" indicates that an individual that receives the compound according to the present invention also receives other cancer therapies, which does not necessarily happen simultaneously, combined in a single pharmacological composition or via the same route of administration. "In combination" therefore refers the treatment of an individual suffering from cancer with more than one cancer therapy. Combined administration encompasses simultaneous treatment, co-treatment or joint treatment, whereby treatment may occur within minutes of each other, in the same hour, on the same day, in the same week or in the same month as one another.

Cancer therapies includes DNA damage inducing therapies, such as irradiation therapy and chemotherapy that work by overwhelming the capacity of the cell to repair DNA damage, resulting in cell death.

In this context, chemotherapy refers to a category of cancer treatment that uses one or more anticancer drugs (chemotherapeutic agents) as part of a standardized chemotherapy regimen. Chemotherapy may be given with a curative intent (which almost always involves combinations of drugs), or it may aim to prolong life or to reduce symptoms (palliative chemotherapy). Chemotherapy is one of the major categories of medical oncology (the medical discipline specifically devoted to pharmacotherapy for cancer and management of patients undergoing cancer treatment). Chemotherapeutic agents are used to treat cancer and are administered in regimens of one or more cycles, combining at least one, preferably two or more agents over a period of days to weeks. Such agents are toxic to cells with high proliferative rates - e.g., to the cancer itself, but also to the GI tract (causing nausea and vomiting), bone marrow (causing various cytopenias) and hair (resulting in baldness), shortness of breathing/lungs (pulmonary embolism, pneumonitis), mucosa and skin toxicities (rashes, dryness), liver (jaundice), kidney (kidney failure), ototoxcity (hearing loss), nerve cells (neurotoxicity causing sensory or motor disturbances) or secondary neoplasia.

Chemotherapeutic agents comprise, without limitation, Actinomycin, All-trans retinoic acid, Azacitidine, Azathioprine, Bleomycin, Bortezomib, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Eribulin, Etoposide, Fluorouracil, Gemcitabine, Hydroxyurea, Idarubicin, Ifosfamid, Imatinib, Irinotecan, Mechlorethamine, Mercaptopurine, Methotrexate, Mitoxantrone, nab-Paclitaxel, Oxaliplatin, Paclitaxel, PEG-liposomales Doxorubin, Pemetrexed, Teniposide, Tioguanine, Topotecan, Valrubicin, Vinblastine, Vincristine, Vindesine, Vinorelbine. Furthermore, chemotherapies comprise the administration of chemotherapeutics agents including, but not limited to anthracyclines such as Daunorubicin, Doxorubicin, Epirubicin, Idarubicin, Valrubicin, Mitoxantrone; Inhibitors of topoisomerase I such as Irinotecan (CPT-11) and Topotecan; Inhibitors of topoisomerase II including Etoposide, Teniposide and Tafluposide; Platinum-based agents such as Carboplatin, Cisplatin and Oxaliplatin; and other chemotherapies such as Bleomycin.

In the context of the present invention, chemotherapy comprises endocrine therapy, including, without limitation, administration of CDK4/6 inhibitors, tamoxifen, aromatase inhibitors, GNRH analoges, plus Everolimus. Furthermore, chemotherapy includes administration of a Her2-targeted therapy, which includes, without limitation, Pertuzumab, Trastuzumab, Lapatinib, and Trastumzumab-Emtansin (Herceptin^{®}, Ontruzant ^{®}, Kadcyla^{®},..).

Furthermore, as used herein, the term chemotherapy comprises PARP inhibitors, such as, without limitation, Rucaparib, Niraparib, talazoparib, olaparib, or veliparib.

As used herein, the term "chemotherapy" or "administration of chemotherapy" comprises the administration of one or more compounds, in particular also a combination therapy of two or more chemotherapeutic agents.

Irradiation or radiation therapy or radiotherapy in the context of the present invention relates to a therapeutic approach using ionizing or ultraviolet-visible (UV/Vis) radiation, generally as part of cancer treatment to control or kill malignant cells such as cancer cells or tumor cells. Radiation therapy may be curative in a number of types of cancer, if they are localized to one area of the body. It may also be used as part of adjuvant therapy, to prevent tumor recurrence after surgery to remove a primary malignant tumor (for example, early stages of breast cancer). Radiation therapy is synergistic with chemotherapy, and can been used before, during, and after chemotherapy in susceptible cancers, preferably during or after, most preferably after chemotherapy. Radiation therapy is commonly applied to the cancerous tumor because of its ability to control cell growth. Ionizing radiation works by damaging the DNA of cancerous tissue leading to cellular death. Radiation therapy can be used systemically or locally for curative, palliative intention or symptomatic control of local tumor burden

Radiation therapy works by damaging the DNA of cancerous cells. This DNA damage is caused by one of two types of energy, photon or charged particle. This damage is either direct or indirect ionization of the atoms which make up the DNA chain. Indirect ionization happens as a result of the ionization of water, leading to the formation of free radicals, including hydroxyl radicals, which then damage the DNA. In photon therapy, most of the radiation effect is mediated through free radicals. Cells have mechanisms for repairing single-strand DNA damage and double-stranded DNA damage. However, double-stranded DNA breaks are much more complex to repair and can lead to dramatic chromosomal abnormalities and genetic deletions, if repaired incorrectly. Targeting double-stranded breaks increases the probability that cells will undergo cell death due to the drastic accumulation of deleterious DNA changes.

The amount of radiation used in photon radiation therapy is measured in gray (Gy), and varies depending on the type and stage of cancer being treated. For curative cases, the typical dose for a solid epithelial tumor ranges from 60 to 80 Gy, while lymphomas are treated with 20 to 40 Gy. Preventive (adjuvant) doses are typically around 45-60 Gy in 1.8-2 Gy fractions (in particular for breast, head, and neck cancers.)

Different types of radiation therapy are known such as external beam radiation therapy, including conventional external beam radiation therapy, stereotactic radiation (radiosurgery), virtual simulation, 3-dimensional conformal radiation therapy, and intensity-modulated radiation therapy, intensity-modulated radiation therapy (IMRT), volumetric modulated arc therapy (VMAT), Particle therapy, auger therapy, brachytherapy, intraoperative radiotherapy, radioisotope therapy and deep inspiration breath-hold. External beam radiation therapy comprises X-ray, gamma-ray and charged particles and can be applied as a low-dose rate or high dose rate depending on the overall therapeutic approach. In internal radiation therapy radioactive substance can be bound to one or more monoclonal antibodies. For example, radioactive iodine can be used for thyroid malignancies. Brachytherapy of High dose regime (HDR) or low dose regime (LDR) can be combined with IR in prostate cancer.

As used herein, "cancer immunotherapy" comprises any kind of therapeutic approach or treatment directed against a tumor employing means of the immune system to negate or destroy tumor material. This includes, without limitation, immune checkpoint modulators, immune cell therapy, adoptive transfer of immune cells or other cells that modulate the immune response, modulation of the immune cells by small molecules or biopharmaceuticals such as monoclonal antibodies, cytokines, chemokines, and cancer treatment vaccines. Therefore, immunotherapy is to be understood in the context of the present invention to encompass a therapeutic agent that uses the immune system to treat cancer. Immunotherapy encompasses, without limitation, cellular and biopharmaceutical and antibody therapy as well as the use of small molecules inducing a modification of the immune response to tumors.

Furthermore, cancer therapies include PARP inhibitors, Bevacizumab and/or modulators of HGF signaling.

PARP inhibitors are a group of pharmacological inhibitors of the enzyme poly ADP ribose polymerase (PARP). They are developed for multiple indications, including the treatment of cancer. Several forms of cancer are more dependent on functional PARP than somatic, germ, i.e. 'non-cancerous', cells, making PARP enzyme family (PARP1, PARP2 etc) an attractive target for cancer therapy. PARP inhibitors appear to improve progression-free survival in women with primary and recurrent platinum-sensitive ovarian cancer that have a partial or complete response to platinum-based chemotherapy, as evidenced mainly by olaparib therapy commenced after conventional treatment.

Bevacizumab, sold under the brand name Avastin, is a medication used to treat a number of types of cancers. For cancer it is given by slow injection into a vein and used for colon cancer, lung cancer, glioblastoma, breast cancer, ovarian cancer and renal-cell carcinoma among others, and can also be used as a cancer treatment in the context of the present invention. Bevacizumab is a monoclonal antibody that mainly functions as an angiogenesis inhibitor. It works by slowing the growth of new blood vessels by inhibiting vascular endothelial growth factor A (VEGF-A), in other words anti-VEGF therapy.

Modulators of HGF signaling include MET kinase inhibitors, HGF inhibitors, Decoy MET, and Immunotherapy targeting MET.

The tyrosine kinase activity of c-MET transduces the mitogenic and motogenic signals elicited by HGF. c-Met is also called tyrosine-protein kinase Met or hepatocyte growth factor receptor (HGFR), is a protein that in humans is encoded by the MET gene. The protein possesses tyrosine kinase activity. The primary single chain precursor protein is post-translationally cleaved to produce the alpha and beta subunits, which are disulfide linked to form the mature receptor. its signaling and structure has been reviewed (for example Gianluca Baldanzi et al. Biomedicines. 2015 Mar; 3(1): 1-31).

Since tumor invasion and metastasis are the main cause of death in cancer patients, interfering with MET signaling appears to be a promising therapeutic approach.

c-Met inhibitors are a class of small molecules that inhibit the enzymatic activity of the c-Met tyrosine kinase, the receptor of hepatocyte growth factor/scatter factor (HGF/SF). These inhibitors may have therapeutic application in the treatment of various types of cancers.

c-Met Kinase inhibitors are low molecular weight molecules that prevent ATP binding to MET, thus inhibiting receptor trans/autophosphorylation and recruitment of the downstream effectors. The limitations of kinase inhibitors include the facts that they only inhibit kinase-dependent MET activation, and that none of them is fully specific for MET. K252a (Fermentek Biotechnology) is a staurosporine analogue isolated from Nocardiopsis sp. soil fungi, and it is a potent inhibitor of all receptor tyrosine kinases (RTKs). At nanomolar concentrations, K252a inhibits both the wild type and the mutant (M1268T) MET function. SU11274 (SUGEN) specifically inhibits MET kinase activity and its subsequent signaling. SU11274 is also an effective inhibitor of the M1268T and H1112Y MET mutants, but not the L1213V and Y1248H mutants. SU11274 has been demonstrated to inhibit HGF-induced motility and invasion of epithelial and carcinoma cells. PHA-665752 (Pfizer) specifically inhibits MET kinase activity, and it has been demonstrated to represses both HGF-dependent and constitutive MET phosphorylation. Furthermore, some tumors harboring MET amplifications are highly sensitive to treatment with PHA-665752. ARQ197 (ArQule) is a promising selective inhibitor of MET, which entered a phase 2 clinical trial in 2008. Foretinib (XL880, Exelixis) targets multiple receptor tyrosine kinases (RTKs) with growth-promoting and angiogenic properties. The primary targets of foretinib are MET, VEGFR2, and KDR. Foretinib has completed a phase 2 clinical trials with indications for papillary renal cell carcinoma, gastric cancer, and head and neck cancer. SGX523 (SGX Pharmaceuticals) specifically inhibits MET at low nanomolar concentrations. MP470 (SuperGen) is a novel inhibitor of c-KIT, MET, PDGFR, Flt3, and AXL. Phase I clinical trial of MP470 had been announced in 2007.

Since HGF is the only known ligand of MET, blocking the formation of a HGF:MET complex blocks MET biological activity. For this purpose, truncated HGF, anti-HGF neutralizing antibodies, and an uncleavable form of HGF have been utilized so far. The major limitation of HGF inhibitors is that they block only HGF-dependent MET activation. NK4, also known as interleukin-32 or natural killer cells protein 4, competes with HGF as it binds MET without inducing receptor activation, thus behaving as a full antagonist. NK4 is a molecule bearing the N-terminal hairpin and the four kringle domains of HGF. Moreover, NK4 is structurally similar to angiostatins, which is why it possesses anti-angiogenic activity. Neutralizing anti-HGF antibodies were initially tested in combination, and it was shown that at least three antibodies, acting on different HGF epitopes, are necessary to prevent MET tyrosine kinase activation. More recently, it has been demonstrated that fully human monoclonal antibodies can individually bind and neutralize human HGF, leading to regression of tumors in mouse models. Two anti-HGF antibodies are currently available: the humanized AV299 (AVEO), and the fully human AMG102 (Amgen). Uncleavable HGF is an engineered form of pro-HGF carrying a single amino-acid substitution, which prevents the maturation of the molecule. Uncleavable HGF is capable of blocking MET-induced biological responses by binding MET with high affinity and displacing mature HGF. Moreover, uncleavable HGF competes with the wild-type endogenous pro-HGF for the catalytic domain of proteases that cleave HGF precursors. Local and systemic expression of uncleavable HGF inhibits tumor growth and, more importantly, prevents metastasis.

Decoy MET refers to a soluble truncated MET receptor. Decoys are able to inhibit MET activation mediated by both HGF-dependent and independent mechanisms, as decoys prevent both the ligand binding and the MET receptor homodimerization. CGEN241 (Compugen) is a decoy MET that is highly efficient in inhibiting tumor growth and preventing metastasis in animal models.

Drugs used for immunotherapy can act either passively by enhancing the immunologic response to MET-expressing tumor cells, or actively by stimulating immune cells and altering differentiation/growth of tumor cells. Administering monoclonal antibodies (mAbs) is a form of passive immunotherapy. MAbs facilitate destruction of tumor cells by complement-dependent cytotoxicity (CDC) and antibody-dependent cell-mediated cytotoxicity (ADCC). In CDC, mAbs bind to specific antigen, leading to activation of the complement cascade, which in turn leads to formation of pores in tumor cells. In ADCC, the Fab domain of a mAb binds to a tumor antigen, and Fc domain binds to Fc receptors present on effector cells (phagocytes and NK cells), thus forming a bridge between an effector and a target cells. This induces the effector cell activation, leading to phagocytosis of the tumor cell by neutrophils and macrophages. Furthermore, NK cells release cytotoxic molecules, which lyse tumor cells. DN30 is monoclonal anti-MET antibody that recognizes the extracellular portion of MET. DN30 induces both shedding of the MET ectodomain as well as cleavage of the intracellular domain, which is successively degraded by proteasome machinery. As a consequence, on one side MET is inactivated, and on the other side the shed portion of extracellular MET hampers activation of other MET receptors, acting as a decoy. DN30 inhibits tumor growth and prevents metastasis in animal models. OA-5D5 is one-armed monoclonal anti-MET antibody that was demonstrated to inhibit orthotopic pancreatic and glioblastoma tumor growth and to improve survival in tumor xenograft models. OA-5D5 is produced as a recombinant protein in Escherichia coli. It is composed of murine variable domains for the heavy and light chains with human IgG1 constant domains. The antibody blocks HGF binding to MET in a competitive fashion. Active immunotherapy to MET-expressing tumors can be achieved by administering cytokines, such as interferons (IFNs) and interleukins (IL-2), which triggers non-specific stimulation of numerous immune cells. IFNs have been tested as therapies for many types of cancers and have demonstrated therapeutic benefits. IL-2 has been approved by the U.S. Food and Drug Administration (FDA) for the treatment of renal cell carcinoma and metastatic melanoma, which often have deregulated MET activity.

In the context of the invention, the determined HGF level can be indicative of initiating or continuing a routine treatment, for example in case of a low severity level of HGF, or can be indicative of modifying a routine treatment, either before or after initiation of such a routine treatment. As used herein, the term routine treatment or standard treatment relates to treatment regimen that the treating physician would initiate upon diagnosis of the cancer in the patient under conditions, where the method of the invention is not applied due to routine considerations in the respective medical institution (such as the application of established standard operating procedures (SOPs)) and considering the specifics of the individual case. Such a standard/routine treatment that is initiated upon diagnosis may also be referred to as a "first line therapy", commonly summarized in national guidelines, such as (but not exclusively) NICE, S3 guidelines, ASCO.

In general, the term "first line therapy" refers to the treatment regimen or regimens that are generally accepted by the medical establishment/a medical institution in charge of treating a patient for initial treatment of a given type and stage of cancer. Herein, it may also be referred to as primary therapy/treatment, standard therapy/treatment, induction therapy/treatment, maintenance therapy/treatment or routine therapy/treatment. The intent of first-line therapy is to cure the cancer if possible. Primary therapy may include the first assault of chemotherapy drugs on a malignancy.

First-line therapies for cancers are usually a combination of one or more of the following: surgery, chemotherapy and radiation. There are different types of these treatment modalities. Variations and tweaks to the therapy regimens may be employed as the doctor takes into account the patient's age, general health, other diseases, urgency of response and stage of cancer.

In general, second-line therapies and in particular second-line chemotherapies are those tried when the first line treatment regimen and the chemotherapeutic agents used therein did not work adequately or could not be administered due to contraindications. The management of a cancer case requires regular evaluation of treatment and adjustment as needed. A break with the primary treatment and an adoption of a new regimen signals "second-line treatment." The first-line therapy (i.e. the routine/standard treatment initiated according to the treating physicians judgement and/or established standard operating procedures of the medical institution in charge of the treatment) may not have worked, may have had some limited efficacy, or may have produced unacceptable side effects.

In the context of the present invention, the necessity to deviated from an ongoing first line treatment, wherein the initial routine treatment is modified or discontinued to switch to a different (second line) treatment regimen, can be indicated by the HGF level, which may be above a certain threshold value at a given time point at and/or after diagnosis. In embodiments, an HGF level determined before initiation of a primary treatment can be indicative of modifying a usual routine (first-line) treatment from the beginning.

Sometimes first-line therapies show progress for a period of time followed by a stalling or continued growth of the cancer. Certain drugs are specifically approved by regulatory authorities as second-line therapy. This labeling is common for new drugs that treat cancers which already have accepted treatments. The idea is that if the older drugs and regimens used don't work, there is less to lose by using the new drug. After years of experience and many patients treated with the new drug, the manufacturer may ask the FDA to expand the labeling to include first-line treatment and/or treatment on other forms of cancer. However, treating physicians have the freedom to use these drugs from the beginning, for example if initiation of a routine first-line treatment would not be indicated by the HGF level determined in the context of the method of the invention.

In the context of the present invention, the determined HGF level can be indicative of the use of second-line therapies from the beginning/as an initial treatment (for example, when the HGF level at the time point of diagnosis is indicative thereof, such as an HGF level above a threshold level). Furthermore, if during an ongoing primary standard treatment (routine first-line therapy) an HGF level indicative of an adverse event is determined in a sample of the patient, this can be indicative of a change of the ongoing treatment regimen, which may include the use of second-line therapies, such as second-line chemotherapy.

In the context of the present invention, a modification of the treatment can involve a change in the dose of a therapeutic measure, such as a drug or radiation, a different route of administration or a different administration regimen, such as a prolonged time of administration, or other parameters of the respective therapeutic measures. Furthermore, in can involve the administration of additional or alternative therapeutic measures, such as the administration of an additional drug in addition to drugs that have already been administered. Furthermore, certain measures that appear to be unsuccessful may be discontinued and other measures may be applied. Accordingly, a modification can be an addition or a replacement of therapeutic measures.

In the context of the invention, a modification of a routine treatment may include the administration of PARP inhibitors, Bevacizumab and/or modulators of HGF signaling in patients, where the use of these therapeutics would not be a routine treatment.

Ovarian cancer is a cancer that forms in or on an ovary. As used herein, the term ovarian cancer comprises of ovarian, fallopian tube and peritoneal cancer. It results in abnormal cells that have the ability to invade or spread to other parts of the body to form metastasis. Often, spreading of cancer cells already begins at a stage where there are no or only vague symptoms, which only become more noticeable as the cancer progresses. Symptoms may include bloating, pelvic pain, abdominal swelling, and loss of appetite, among others. Common areas to which the cancer may spread include the lining of the abdomen, lymph nodes, lungs, and liver. The risk of ovarian cancer increases in women who have ovulated more over their lifetime, such as women who have never had children, women who begin ovulation at a younger age and women who reach menopause at an older age. Further risk factors include hormone therapy after menopause, fertility medication, and obesity. Factors that decrease risk include hormonal birth control, bilateral salpingectomy and/or oophorectomy and breast feeding. About 10% of cases of ovarian cancer are related to inherited genetic risk; women with mutations in the genes BRCA1 or BRCA2 have about a 50% chance of developing the disease, as compared to about 2% risk of that cancer in the entire population. Ovarian carcinoma is the most common subtype of ovarian cancer, comprising about 95% of cases. Treatment usually includes some combination of surgery, and/or chemotherapy. Outcomes depend on the extent of the disease, the subtype of cancer present, and other medical conditions. The overall five-year survival rate in the United States is 49% (43% in Germany). Outcomes are worse in the developing world.

Early signs and symptoms of ovarian cancer may be absent or subtle. In many cases, symptoms exist for several months before being recognized and diagnosed. Symptoms can be misdiagnosed as irritable bowel syndrome. The early stages of ovarian cancer tend to be painless and symptoms can vary based on the subtype. Ovarian borderline tumors, also known as low malignant potential (LMP) ovarian tumors, do not cause an increase in CA125 levels and are not identifiable with an ultrasound. The typical symptoms of an LMP tumor can include abdominal distension or pelvic pain. Particularly large masses tend to be benign or borderline. The most typical symptoms of ovarian cancer include bloating, increased abdominal circumference, abdominal or pelvic pain or discomfort, back pain, irregular menstruation or postmenopausal vaginal bleeding, pain or bleeding after or during sexual intercourse, loss of appetite, fatigue, diarrhea, indigestion, heartburn, constipation or diarrhea, nausea, feeling full, and possibly urinary symptoms (including frequent urination and urgent urination). In later stages, the growing mass may cause pain if ovarian torsion develops. Symptoms can be caused by a mass pressing on the other abdominopelvic organs or from metastases. If these symptoms start to occur more often or more severely than usual, especially after no significant history of such symptoms, ovarian cancer is considered. There is no recommended screening program for ovarian cancer - however there are efforts aiming to establish this, such as the UKCTOCS trial).

Diagnosis of ovarian cancer usually starts with a full medical history, including family history, physical examination (including a pelvic examination), a blood test (for CA-125 and sometimes other markers), and transvaginal ultrasound. Sometimes a rectovaginal examination is used to help plan surgery. The diagnosis must be confirmed by tissue biopsy and histology. This can be achieved by surgery, abdominal or pleural fluid analysis and or other tissue biopsies at sites of suspected tumor/metastatic masses.

In embodiments, the present invention relates to primary epithelial ovarian cancer, including peritoneal cancer and fallopian tube cancer, also known as ovarian epithelial carcinoma, which is the most is the most common type of ovarian cancer, representing approximately 90% of ovarian cancers. It includes serous tumor, endometrioid tumor, and mucinous cystadenocarcinoma. Less common comprised tumors are malignant Endometrioid ovarian cancer, Clear cell ovarian cancer, and Brenner tumor (transitional cell carcinoma of the ovary). Epithelial ovarian cancers develop from the epithelium, a layer of cells that covers the ovary and the fallopian tube. It is thought that the majority of high-grade serous ovarian cancer (the most common subtype of epithelial ovarian cancer with the worst prognosis) originates from the epithelial lining of the fallopian tube (Klotz and Wimberger, et al. 2017)

Ovarian cancer is staged using the FIGO staging system and uses information obtained after primary surgery, which can include a total abdominal hysterectomy via midline laparotomy, removal of (usually) both ovaries and Fallopian tubes, (usually) the omentum, pelvic (peritoneal) washings, assessment of retroperitoneal lymph nodes (including the pelvic and para-aortic lymph nodes), appendectomy in suspected mucinous tumors, and pelvic/peritoneal biopsies for cytopathology. Around 30% of ovarian cancers that appear confined to the ovary have metastasized microscopically, which is why even stage-I cancers must be staged completely. 22% of cancers presumed to be stage I are observed to have lymphatic metastases.

The AJCC staging system describes the extent of the primary tumor (T), the absence or presence of metastasis to nearby lymph nodes (N), and the absence or presence of distant metastasis (M). The most common stage at diagnosis is stage IIIc, with over 70% of diagnoses.

**Table 1. FIGO stages of ovarian cancer.**

| **Stage** | | | | **Description** |
|---|---|---|---|---|
| I | | | | Cancer is completely limited to the ovary |
| | IA | | | involves one ovary, capsule intact, no tumor on ovarian surface, negative washings |
| | IB | | | involves both ovaries; capsule intact; no tumor on ovarian surface; negative washings |
| | IC | | | tumor involves one or both ovaries |
| | IC1 | | | surgical spill |
| | IC2 | | | capsule has ruptured or tumor on ovarian surface |
| | IC3 | | | positive ascites or washings |
| II | | | | pelvic extension of the tumor (must be confined to the pelvis) or primary peritoneal tumor, involves one or both ovaries |
| | IIA | | | tumor found on uterus or fallopian tubes |
| | IIB | | | tumor elsewhere in the pelvis |
| III | | | | cancer found outside the pelvis or in the retroperitoneal lymph nodes, involves one or both ovaries |
| | IIIA | | | metastasis in retroperitoneal lymph nodes or microscopic extrapelvic metastasis |
| | | IIIA1 | | metastasis in retroperitoneal lymph nodes |
| | | | IIIA1 (i) | the metastasis is less than 10 mm in diameter |
| | | | IIIA1 (ii) | the metastasis is greater than 10 mm in diameter |
| | | IIIA2 | | microscopic metastasis in the peritoneum, regardless of retroperitoneal lymph node status |
| | IIIB | | | metastasis in the peritoneum less than or equal to 2 cm in diameter, regardless of retroperitoneal lymph node status; or metastasis to liver or spleen capsule |
| | IIIC | | | metastasis in the peritoneum greater than 2 cm in diameter, regardless of retroperitoneal lymph node status; or metastasis to liver or spleen capsule |
| IV | | | | distant metastasis (i.e. outside of the peritoneum) |
| | IVA | | | pleural effusion containing cancer cells |
| | IVB | | | metastasis to distant organs (including the parenchyma of the spleen or liver), or metastasis to the inguinal and extra-abdominal lymph nodes |

The AJCC staging system describes the extent of the primary tumor (T), the absence or presence of metastasis to nearby lymph nodes (N), and the absence or presence of distant metastasis (M). The AJCC system can be correlated with the FIGO system.

Grade 1 ovarian cancers/tumors have well differentiated cells (look very similar to the normal tissue) and are the ones with the best prognosis. Grade 2 tumors are also called moderately well-differentiated and they are made up of cells that resemble the normal tissue. Grade 3 tumors have the worst prognosis and their cells are abnormal, referred to as poorly differentiated.

Metastasis in ovarian cancer is very common in the abdomen, and occurs via exfoliation, where cancer cells burst through the ovarian capsule and are able to move freely throughout the peritoneal cavity. Ovarian cancer metastases usually grow on the surface of organs rather than the inside; they are also common on the omentum and the peritoneal lining. Cancer cells can also travel through the lymphatic system and metastasize to lymph nodes connected to the ovaries via blood vessels; i.e. the lymph nodes along the infundibulopelvic ligament, the broad ligament, and the round ligament. The most commonly affected groups include the paraaortic, hypogastric, external iliac, obturator, and inguinal lymph nodes. Usually, ovarian cancer does not metastasize to the liver, lung, brain, or kidneys unless it is recurrent disease; this differentiates ovarian cancer from many other forms of cancer.

Once it is determined that ovarian cancer is present, treatment is scheduled by a gynecologic oncologist. Treatment usually involves surgery and chemotherapy (in particular administration of platin- and/or taxan-based chemotherapy agents), and sometimes radiotherapy, regardless of the subtype of ovarian cancer (standard/routine treatment).

Surgical treatment may be sufficient for well-differentiated malignant tumors and confined to the ovary. Addition of chemotherapy may be required for more aggressive tumors confined to the ovary. For patients with advanced disease, a combination of surgical reduction with a combination chemotherapy regimen is standard.

Surgery has been the standard of care for decades and may be necessary in obtaining a specimen for confirming diagnosis. The surgery depends upon the extent of nearby invasion of other tissues by the cancer when it is diagnosed. This extent of the cancer is described by assigning it a stage, the presumed type, and the grade of cancer. The gynecological surgeon may remove one (unilateral oophorectomy; almost only in low-grade or borderline tumors) or both ovaries (bilateral oophorectomy). The Fallopian tubes (salpingectomy), uterus (hysterectomy), peritoneum (peritonectomy) and the omentum (omentectomy) may also be removed. Typically, all of these organs are removed. This may also include bulky lymph nodes and/or pelvic/paraaortic lymph nodes (depending on tumor stage, lymphadenectomy), the spleen (splenectomy), or affected lobes of the liver (partial hepatectomy).

In advanced ovarian cancers, where complete macroscopic resection is not an option, as much tumor as possible is removed in a procedure called surgical debulking. This surgery is not always successful and is less likely to be successful in women with extensive metastases in the peritoneum, stage- IV disease, cancer in the transverse fissure of the liver, mesentery, or diaphragm, and large areas of ascites. However, if complete macroscopic resection cannot be achieved, surgery may not be indicated. Sometimes complete macroscopic resection cannot be achieved despite best efforts, but this cannot be known before the surgical procedure.

Chemotherapy has been a general standard of care for ovarian cancer for decades, although with variable protocols. Chemotherapy is used after surgery to treat any residual or microscopic disease, if appropriate, and such additional chemotherapy is also called "adjuvant chemotherapy". In some cases, there may be reason to perform chemotherapy first, followed by surgery. This is called "neoadjuvant chemotherapy", and is common when there is a contraindication to perform surgery at first, a reduction in tumor mass due to chemotherapy will result in a reduction of tumor mass and increase the chances of a complete macroscopic resection. Though it has not been shown to increase survival, it can reduce the risk of complications after surgery.

Adjuvant chemotherapy is used in stage 1 cancer typically if the tumor is of a high histologic grade (grade 3) or the highest substage (stage 1c), provided the cancer has been optimally staged during surgery. Adjuvant therapy is used for ovarian cancers staged FIGO IIB or above. Bevacizumab may be started from the 2^{nd} cycle of adjuvant platinum-based-chemotherapy for cancers with the stages IIIB, IIIC and IV. Adjuvant chemotherapy has been found to improve survival and reduce the risk of ovarian cancer recurring compared to no adjuvant therapy in women with early stage epithelial ovarian cancer.

Chemotherapy in ovarian cancer typically consists of platinum-based regimes, combined with non-platinum-based chemotherapy. Common therapies can include paclitaxel, cisplatin, topotecan, doxorubicin, epirubicin, and gemcitabine. Carboplatin is typically given in combination with either paclitaxel or docetaxel; the typical combination is carboplatin with paclitaxel. Carboplatin is superior to cisplatin in that it is less toxic and has fewer side effects, generally allowing for an improved quality of life in comparison, though both are similarly effective. Three-drug regimens have not been found to be more effective, and platins alone or nonplatins alone are less effective than platins and nonplatins in combination. There is a small benefit in platinum-based chemotherapy compared with non-platinum therapy. Platinum combinations can offer improved survival over single platinum. In people with relapsed ovarian cancer, evidence suggests topotecan has a similar effect on overall survival as paclitaxel and topotecan plus thalidomide, whilst it is superior to treosulfan and not as effective as pegylated liposomal doxorubicin in platinum-sensitive people. However, treatment of recurring ovarian cancer depends on many aspects, such as time point of recurrence. In some cases, recurring cancer could be treated with platinum-based chemotherapy again.

In ovarian cancer, chemotherapy can be given intravenously and/or in the peritoneal cavity. Though intraperitoneal chemotherapy is associated with longer progression-free survival and overall survival, it also causes more adverse side effects than intravenous chemotherapy and is mainly used when the cancer has been optimally debulked. Intraperitoneal chemotherapy can be highly effective because ovarian cancer mainly spreads inside the peritoneal cavity, and higher doses of the drugs can reach the tumors this way.

If ovarian cancer progresses or recurs after initial standard/routine treatment as determined by the treating physician based on the individual case of the patient, this is referred to as cancer progression or cancer recurrence. A recurring ovarian cancer is commonly considered partially platinum-sensitive or platinum-resistant, based on the time since the last recurrence treated with platins: partially platinum-sensitive cancers recurred 6-12 months after last treatment, and platinum-resistant cancers have an interval of less than 6 months. In case of ovarian cancer recurrence, second-line chemotherapy may be administered.

For platinum-sensitive tumors, platinums are typically the drugs of choice for second-line chemotherapy, in combination with other cytotoxic agents. Regimens include carboplatin combined with pegylated liposomal doxorubicin, gemcitabine, or to a lesser degree paclitaxel. Carboplatin-doublet therapy can be combined with paclitaxel for increased efficacy in some cases. Another potential additional maintenance therapy for platinum-sensitive recurrences is the use of PARP inhibitors after completion and (at least partial) response to second-line platinum-based chemotherapy. These PARP inhibitors include olaparib, niraparib or rucaparib, which may improve progression-free survival but has not been shown to improve overall survival, independent of biomarker status. Olaparib, niraparib, rucaparib are approved for recurrent platinum-sensitive ovarian cancer. Olaparib was approved by the US FDA for use in BRCA-associated ovarian cancer that had previously been treated with chemotherapy and may be used as a second-line chemotherapy also in the context of the present invention. For recurrent germ cell tumors, an additional 4 cycles of BEP chemotherapy is often used for those who have been treated with surgery and/or platins.

If the tumor is determined to be platinum-resistant, topotecan, dactinomycin, and cyclophosphamide (VAC) or some combination of paclitaxel and gemcitabine may be used as a second-line therapy. For platinum-resistant tumors, single-drug regimens of paclitaxel, topotecan, trabectedin, treosulfan, pegylated liposomal doxorubicin, etoposide, or gemcitabine may be used.

Novel chemotherapeutic agents for who develop resistance to chemotherapy drugs are constantly being developed, for example to inhibit the development of new blood vessels (angiogenesis) or immunomodulators (checkpoint inhibitors), and these may represent useful second-line chemotherapy (as monotherapy or in combination) in the context of the invention.

In stage 1c and 2 ovarian cancers, radiation therapy can be used after surgery if there is the possibility of residual disease in the pelvis but the abdomen is cancer-free. Radiotherapy can be used in palliative care of advanced cancers to treat specific symptomatic lesions. A typical course of radiotherapy for ovarian cancer is 5 days a week for 3-4 weeks.

According to the present invention, the term "indicate" in the context of "indicative of a subsequent adverse event" is intended as a measure of risk and/or likelihood. Preferably, the "indication" of the presence or absence of an adverse event is intended as a risk assessment and is typically not to be construed in a limiting fashion as to point definitively to the absolute presence or absence of said event. Therefore, the term "indicative of the absence of a subsequent adverse event" or "indicative of a subsequent adverse event" can be understood as indicating a low or high risk of the occurrence of an adverse event, respectively. In some embodiments a low risk relates to a lower risk compared to HGF levels detected above a certain threshold value. In some embodiments a high risk relates to a higher risk compared to HGF levels detected below a certain threshold value. In the context of "indicates modifying a routine treatment" the term is intended as a measure of likelihood of success of the routine treatment. Preferably, the "indication" of the modification or change of the routine treatment being required is intended to refer to an increased likelihood that the routine treatment is not successful in improving or stabilizing the health status of the patient. Therefore, a modification of the treatment should be considered to prevent the occurrence of an adverse event in the patient.

Hepatocyte growth factor (HGF), or also called scatter factor (SF), is a paracrine cellular growth, motility and morphogenic factor. It is secreted by mesenchymal cells and targets and acts primarily upon epithelial cells and endothelial cells, but also acts on hematopoietic progenitor cells and T cells. It has been shown to have a major role in embryonic organ development, specifically in myogenesis, in adult organ regeneration, and in wound healing. HGF activates a tyrosine kinase signaling cascade after binding to the receptor tyrosine kinase c-Met. Its ability to stimulate mitogenesis, cell motility, and matrix invasion gives it a central role in angiogenesis, tumorogenesis, and tissue regeneration. The gene MET is a proto-oncogene. It is secreted as a single inactive polypeptide and is cleaved by serine proteases into a 69-kDa alpha-chain and 34-kDa beta-chain. A disulfide bond between the alpha and beta chains produces the active, heterodimeric molecule. The protein belongs to the plasminogen subfamily of S1 peptidases but has no detectable protease activity.

Hepatocyte growth factor has been shown to interact with the receptor tyrosine kinase c-Met, identified as the HGF receptor (HGFR). Both overexpression of the Met/HGFR receptor protein and autocrine activation of Met/HGFR by simultaneous expression of the hepatocyte growth factor ligand have been implicated in oncogenesis. Hepatocyte growth factor interacts with the sulfated glycosaminoglycans heparan sulfate and dermatan sulfate. The interaction with heparan sulfate allows hepatocyte growth factor to form a complex with c-Met that is able to transduce intracellular signals leading to cell division and cell migration.

Excessive local expression of HGF in the breasts has been implicated in macromastia. HGF is also importantly involved in normal mammary gland development. HGF has been implicated in a variety of cancers, including of the lungs, pancreas, thyroid, colon, and breast. Increased expression of HGF has been associated with the enhanced and scarless wound healing capabilities of fibroblast cells isolated from the oral mucosa tissue.

HGF was originally identified as a soluble factor promoting hepatocyte growth and liver regeneration. In a parallel way a Scatter Factor (SF) was identified as cytokine secreted by fibroblast promoting dissociation and motility of epithelial cells in culture. Molecular cloning demonstrated that HGF and SF are the same growth factor produced by cells of mesenchymal origin and promoting migration and proliferation depending on the epithelial cell targeted. The HGF receptor (MET) is a prototypal tyrosine kinase receptor which plays a key role in the interaction between mesenchyme and epithelia during embryogenesis and tissue homeostasis. The observation that MET is a proto-oncogene and that its signaling is often subverted in cancer has promoted deep investigations on its molecular structure and signaling proprieties.

The HGF gene map to chromosome 7q21.11, in the same chromosomal region of its receptor MET. By alternative splicing the gene give rise to several transcripts beside the full length isoform (728 aa corresponding to 83 kDa, increased to 92 kDa after glycosylation). The full length pre-pro-HGF feature a strong sequence and structural homology to plasminogen, presenting a signal peptide for secretion (residues 1-31), an amino-terminal heparin binding domain (residues 37-123), 4 kringle domains (residues 128-206, 211-288, 305-383 and 391-469) and a serine protease-like domain (residues 495-721). A close homolog of HGF is macrophage stimulating protein (MSP) with a 45% identity and a similar organization with 4 kringle domains. Two other HGF splicing isoforms have been extensively characterized consisting of the amino-terminal domain linked in tandem with, respectively, the first one (NK1, 24 kDa) or the first two (NK2, 36 kDa) kringle domains. All three isoforms bind to the receptor tyrosine kinase MET and evoke signaling but with different affinity and potency. Indeed, NK1 stimulates cell proliferation, migration and tubular morphogenesis, though at reduced potency and with greater heparan sulfate (HS) dependence compared to full-length HGF, suggesting that the primary MET binding site is contained within this fragment. Conversely, NK2 acts as a competitive inhibitor of full length HGF promoted mitogenicity but retains motogenic activity in vitro and in vivo.

All HGF isoforms are synthesized as pre-peptides that undergo proteolytic cleavage at residue 31 to remove the leader sequence. Full-length pro-HGF also undergoes extensive glycosylation (N-linked at residue 294, 402, 566 and 653, O-linked at residue 476), which is dispensable for biological activity but promotes secretion. Full length HGF requires proteolytic cleavage at the beginning of the serine protease like domain (between R494 and V495) to become a biologically active heterodimer consisting of disulfide-linked α (residues 32-494, 69 kDa after glycosylation) and β (residues 495-728, 32-34 kDa depending on the extension of glycosylation) chains.

As used herein, the term HGF includes several orthologs, including the proteins expressed from the following human genes, mRNAs and according to the corresponding protein sequences defined as follows: Ensembl ENSG00000019991; UniProt P14210; NCBI mRNA reference sequences NM_000601, NM_001010931, NM_001010932, NM_001010933, NM_001010934; NCBI protein reference sequences NP_000592, NP_001010931, NP_001010932, NP_001010933, NP_001010934.

In embodiments, HGF relates to a protein with the amino acid sequence according to one of the preferred sequences disclosed in ***Table 8.***

**Table 8. Amino acid sequences HGF according to the present invention.**

| | |
|---|---|
| SEQ ID NO 1: | MWVTKLLPALLLQHVLLHLLLLPIAIPYAEGQRKRRNTIHEFKKSA KTTLIKIDPALKIKTKKVNTADQCANRCTRNKGLPFTCKAFVFDKA RKQCLWFPFNSMSSGVKKEFGHEFDLYENKDYIRNCIIGKGRSY KGTVSITKSGIKCQPWSSMIPHEHSFLPSSYRGKDLQENYCRNP RGEEGGPWCFTSNPEVRYEVCDIPQCSEVECMTCNGESYRGL MDHTESGKICQRWDHQTPHRHKFLPERYPDKGFDDNYCRNPD GQPRPWCYTLDPHTRWEYCAIKTCADNTMNDTDVPLETTECIQG QGEGYRGTVNTIWNGIPCQRWDSQYPHEHDMTPENFKCKDLRE NYCRNPDGSESPWCFTTDPNIRVGYCSQIPNCDMSHGQDCYRG NGKNYMGNLSQTRSGLTCSMWDKNMEDLHRHIFWEPDASKLN ENYCRNPDDDAHGPWCYTGNPLIPWDYCPISRCEGDTTPTIVNL DHPVISCAKTKQLRVVNGIPTRTNIGWMVSLRYRNKHICGGSLIK ESWVLTARQCFPSRDLKDYEAWLGIHDVHGRGDEKCKQVLNVS QLVYGPEGSDLVLMKLARPAVLDDFVSTIDLPNYGCTIPEKTSCS VYGWGYTGLINYDGLLRVAHLYIMGNEKCSQHHRGKVTLNESEI CAGAEKIGSGPCEGDYGGPLVCEQHKMRMVLGVIVPGRGCAIP NRPGIFVRVAYYAKWIHKIILTYKVPQS |
| Amino acid (AA) sequence HGF Isoform 1 (UniProt identifier: P14210-1) | |
| SEQ ID NO 2: | MWVTKLLPALLLQHVLLHLLLLPIAIPYAEGQRKRRNTIHEFKKSA KTTLIKIDPALKIKTKKVNTADQCANRCTRNKGLPFTCKAFVFDKA RKQCLWFPFNSMSSGVKKEFGHEFDLYENKDYIRNCIIGKGRSY KGTVSITKSGIKCQPWSSMIPHEHSFLPSSYRGKDLQENYCRNP RGEEGGPWCFTSNPEVRYEVCDIPQCSEVECMTCNGESYRGL MDHTESGKICQRWDHQTPHRHKFLPERYPDKGFDDNYCRNPD GQPRPWCYTLDPHTRWEYCAIKTCET |
| Amino acid (AA) sequence HGF Isoform 2 (UniProt identifier: P14210-2) | |
| SEQ ID NO 3: | MWVTKLLPALLLQHVLLHLLLLPIAIPYAEGQRKRRNTIHEFKKSA KTTLIKIDPALKIKTKKVNTADQCANRCTRNKGLPFTCKAFVFDKA RKQCLWFPFNSMSSGVKKEFGHEFDLYENKDYIRNCIIGKGRSY KGTVSITKSGIKCQPWSSMIPHEHSYRGKDLQENYCRNPRGEEG GPWCFTSNPEVRYEVCDIPQCSEVECMTCNGESYRGLMDHTES GKICQRWDHQTPHRHKFLPERYPDKGFDDNYCRNPDGQPRPW CYTLDPHTRWEYCAIKTCADNTMNDTDVPLETTECIQGQGEGYR |
| Amino acid (AA) sequence HGF Isoform 3 (UniProt identifier: P14210-3) | |
| | GTVNTIWNGIPCQRWDSQYPHEHDMTPENFKCKDLRENYCRNP DGSESPWCFTTDPNIRVGYCSQIPNCDMSHGQDCYRGNGKNY MGNLSQTRSGLTCSMWDKNMEDLHRHIFWEPDASKLNENYCR NPDDDAHGPWCYTGNPLIPWDYCPISRCEGDTTPTIVNLDHPVIS CAKTKQLRVVNGIPTRTNIGWMVSLRYRNKHICGGSLIKESWVLT ARQCFPSRDLKDYEAWLGIHDVHGRGDEKCKQVLNVSQLVYGP EGSDLVLMKLARPAVLDDFVSTIDLPNYGCTIPEKTSCSVYGWGY TGLINYDGLLRVAHLYIMGNEKCSQHHRGKVTLNESEICAGAEKI GSGPCEGDYGGPLVCEQHKMRMVLGVIVPGRGCAIPNRPGIFV RVAYYAKWIHKIILTYKVPQS |
| SEQ ID NO 4: | MWVTKLLPALLLQHVLLHLLLLPIAIPYAEGQRKRRNTIHEFKKSA KTTLIKIDPALKIKTKKVNTADQCANRCTRNKGLPFTCKAFVFDKA RKQCLWFPFNSMSSGVKKEFGHEFDLYENKDYIRNCIIGKGRSY KGTVSITKSGIKCQPWSSMIPHEHSFLPSSYRGKDLQENYCRNP RGEEGGPWCFTSNPEVRYEVCDIPQCSEVECMTCNGESYRGL MDHTESGKICQRWDHQTPHRHKFLPERYPDKGFDDNYCRNPD GQPRPWCYTLDPHTRWEYCAIKNMRDITWALN |
| Amino acid (AA) sequence HGF Isoform 4 (UniProt identifier: P14210-4) | |
| SEQ ID NO 5: | MWVTKLLPALLLQHVLLHLLLLPIAIPYAEGQRKRRNTIHEFKKSA KTTLIKIDPALKIKTKKVNTADQCANRCTRNKGLPFTCKAFVFDKA RKQCLWFPFNSMSSGVKKEFGHEFDLYENKDYIRNCIIGKGRSY KGTVSITKSGIKCQPWSSMIPHEHSYRGKDLQENYCRNPRGEEG GPWCFTSNPEVRYEVCDIPQCSEVECMTCNGESYRGLMDHTES GKICQRWDHQTPHRHKFLPERYPDKGFDDNYCRNPDGQPRPW CYTLDPHTRWEYCAIKTCET |
| Amino acid (AA) sequence HGF Isoform 5 (UniProt identifier: P14210-5) | |
| SEQ ID NO6: | MWVTKLLPALLLQHVLLHLLLLPIAIPYAEGQRKRRNTIHEFKKSA KTTLIKIDPALKIKTKKVNTADQCANRCTRNKGLPFTCKAFVFDKA RKQCLWFPFNSMSSGVKKEFGHEFDLYENKDYIRNCIIGKGRSY KGTVSITKSGIKCQPWSSMIPHEHSFLPSSYRGKDLQENYCRNP RGEEGGPWCFTSNPEVRYEVCDIPQCSEGK |
| Amino acid (AA) sequence HGF Isoform 6 (UniProt identifier: P14210-6) | |

In one embodiment the invention therefore encompasses a HGF polypeptide as described herein comprising or consisting of an amino acid sequence selected from the group consisting of:
a) an amino acid sequence comprising or consisting of an amino acid sequence according to SEQ ID NO 1-6;
b) an amino acid sequence having sufficient sequence identity to be functionally analogous/equivalent to an amino acid sequence according to a), comprising preferably a sequence identity to an amino acid sequence according to a) of at least 70%, 80%, preferably 90%, more preferably 95%; and
c) an amino acid sequence of a), or b) which is modified by deletions, additions, substitutions, translocations, inversions and/or insertions and functionally analogous/equivalent to an amino acid sequence according to a), or b).

Functionally analogous sequences refer preferably to the ability to encode a functional peptide comprising a homology to a conserved sequence of HGF. Preferably, the conserved sequence of HGF corresponds to an amino acid sequence according to SEQ ID NO 1-6.

In this context, functionality of HGF can be measured by well-established assays of HGF activity known to a person skilled in the art.

In embodiments of the invention, HGF can relate to a polypeptide as described herein comprising or consisting of an amino acid sequence SEQ ID NO 1-6, or variants of these sequences, wherein the sequence variant may comprise a sequence identity to SEQ ID NO 1-6 of 50, 55, 60, 65, 70, 75, 80, 81, 82, 83, 84, 85, 86, 87, 88, 89, 90, 91, 92, 93, 94, 95, 96, 97, 98 or 99%. Sequence identity may be determined using methods known to one skilled in the art, such as BLAST or other sequence alignment tools.

It is also envisaged herein that a peptide and fragment thereof of HGF can be used for the herein described methods. For example, the peptide or the fragment of HGF preferably at least 12 amino acids, preferably more than 20 amino acids, more preferably more than 40 amino acids long.

The determination of these various forms of HGF or fragments thereof also encompass measuring and/or detecting specific sub-regions of these molecules, for example by employing antibodies or other affinity reagents directed against a particular portion of the molecules, or by determining the presence and/or quantity of the molecules by measuring a portion of the protein using mass spectrometry.

Any one or more of the "HGF peptides or fragments" described herein may be employed in the context of the present invention. HGF or a suitable fragment thereof can be performed by any suitable method known in the art for determining Protein-biomarker levels or concentrations in a sample. For example, an Immunoassay-based method such as an ELISA based determining method can be used. Furthermore, determining by mass spectrometry-based methods can be employed. By way of example, a method may be employed selected from the group consisting of mass spectrometry (MS), luminescence immunoassay (LIA), radioimmunoassay (RIA), chemiluminescence- and fluorescence- immunoassays, enzyme immunoassay (EIA), Enzyme-linked immunoassays (ELISA), luminescence-based bead arrays, magnetic beads based arrays, protein microarray assays, rapid test formats such as for instance immunochromatographic strip tests, rare cryptate assay, and automated systems/analyzers, such as but not exclusively flow cytometry. Further, assays suitable for point-of-care testing and rapid test formats such as for instance immune-chromatographic strip tests can be employed. Automated immunoassays are also intended. Combinations between immune assays and nucleic acid-based assays are also envisaged as falling within the scope of the present invention.

Determination of HGF and optionally other markers based on antibody recognition is a preferred embodiment of the invention. As used herein, the term, "antibody" refers to immunoglobulin molecules and immunologically active portions of immunoglobulin (Ig) molecules, i.e., molecules that contain an antigen binding site that specifically binds to an antigen. According to the invention, the antibodies may be monoclonal as well as polyclonal antibodies. Particularly, antibodies that are specifically binding to at least HGF or fragments thereof are used.

Alternatively, instead of antibodies, other capture molecules or molecular scaffolds that specifically and/or selectively recognize HGF may be encompassed by the scope of the present invention. Herein, the term "capture molecules" or "molecular scaffolds" comprises molecules which may be used to bind target molecules or molecules of interest, i.e. analytes (e.g. HGF, or CA125), from a sample. Capture molecules must thus be shaped adequately, both spatially and in terms of surface features, such as surface charge, hydrophobicity, hydrophilicity, presence or absence of lewis donors and/or acceptors, to specifically bind the target molecules or molecules of interest. Hereby, the binding may, for instance, be mediated by ionic, van-der-Waals, pi-pi, sigma-pi, hydrophobic or hydrogen bond interactions or a combination of two or more of the aforementioned interactions or covalent interactions between the capture molecules or molecular scaffold and the target molecules or molecules of interest. In the context of the present invention, capture molecules or molecular scaffolds may for instance be selected from the group consisting of a nucleic acid molecule, a carbohydrate molecule, a PNA molecule, a protein, a peptide and a glycoprotein. Capture molecules or molecular scaffolds include, for example, aptamers, DARpins (Designed Ankyrin Repeat Proteins). Affimers and the like are included.

As used herein, the "detection reagents" for determining the level of HGF in a sample are reagents that are suitable to determine HGF and potentially other markers, such as CA125. Such exemplary detection reagents are, for example, ligands, e.g. antibodies or fragments thereof, which specifically bind to the peptide or epitopes of the herein described marker(s). Such ligands might be used in immunoassays as described above. Further reagents that are employed in the immunoassays to determine the level of the marker(s) may also be comprised in the kit and are herein considered as detection reagents. Detection reagents can also relate to reagents that are employed to detect the markers or fragments thereof by MS based methods. Such detection reagent can thus also be reagents, e.g. enzymes, chemicals, buffers, etc, that are used to prepare the sample for the MS analysis. A mass spectrometer can also be considered as a detection reagent. Detection reagents according to the invention can also be calibration solution(s), e.g. which can be employed to determine and compare the level of the marker(s), often referred to as a standard solution against which the concentration of the samples in question can be determined.

Preferably, in the context of an immunoassay for determining the level of HGF one of the antibodies can be labelled and the other antibody can be bound to a solid phase or can be bound selectively to a solid phase. In a particularly preferred aspect of the assay, one of the antibodies is labelled while the other is either bound to a solid phase or can be bound selectively to a solid phase. The first antibody and the second antibody can be present dispersed in a liquid reaction mixture, and wherein a first labelling component which is part of a labelling system based on fluorescence or chemiluminescence extinction or amplification is bound to the first antibody, and a second labelling component of said labelling system is bound to the second antibody so that, after binding of both antibodies to said HGF or fragments thereof to be detected, a measurable signal which permits detection of the resulting sandwich complexes in the measuring solution is generated. The labelling system can comprise a rare earth cryptate or chelate in combination with a fluorescent or chemiluminescent dye, in particular of the cyanine type.

In one embodiment, the method is executed as heterogeneous sandwich immunoassay, wherein one of the antibodies is immobilized on an arbitrarily chosen solid phase, for example, the walls of coated test tubes (e.g. polystyrol test tubes; coated tubes; CT) or microtiter plates, for example composed of polystyrol, or to particles, such as for instance magnetic particles, whereby the other antibody has a group resembling a detectable label or enabling for selective attachment to a label, and which serves the detection of the formed sandwich structures. A temporarily delayed or subsequent immobilization using suitable solid phases is also possible.

The level of the marker of the present invention, e.g. the HGF or fragments thereof, HGF or fragments thereof, or other markers, can also be determined by a mass spectrometric (MS) based methods. Such a method may comprise detecting the presence, amount or concentration of one or more modified or unmodified fragment peptides of e.g. HGF in said biological sample or a protein digest (e.g. tryptic digest) from said sample, and optionally separating the sample with chromatographic methods, and subjecting the prepared and optionally separated sample to MS analysis. For example, selected reaction monitoring (SRM), multiple reaction monitoring (MRM) or parallel reaction monitoring (PRM) mass spectrometry may be used in the MS analysis, particularly to determine the amounts of HGF or fragments thereof.

Herein, the term "mass spectrometry" or "MS" refers to an analytical technique to identify compounds by their mass. In order to enhance the mass resolving and mass determining capabilities of mass spectrometry, the samples can be processed prior to MS analysis. Accordingly, the invention relates to MS detection methods that can be combined with immuno-enrichment technologies, methods related to sample preparation and/or chromatographic methods, preferably with liquid chromatography (LC), more preferably with high performance liquid chromatography (HPLC) or ultra high performance liquid chromatography (UHPLC). Sample preparation methods comprise techniques for lysis, fractionation, digestion of the sample into peptides, depletion, enrichment, dialysis, desalting, alkylation and/or peptide reduction. However, these steps are optional. The selective detection of analyte ions may be conducted with tandem mass spectrometry (MS/MS). Tandem mass spectrometry is characterized by mass selection step (as used herein, the term "mass selection" denotes isolation of ions having a specified m/z or narrow range of m/z's), followed by fragmentation of the selected ions and mass analysis of the resultant product (fragment) ions.

The cut-off/threshold values disclosed herein refer preferably to measurements of the protein level of HGF or fragments thereof in a serum sample obtained from a patient by means of the Thermo Fisher ELISA Assay (Catalog # BMS2069INST and/or Catalog # KAC2211). Accordingly, the values disclosed herein may vary to some extent depending on the detection/measurement method employed, and the specific values disclosed herein are intended to also read on the corresponding values determined by other methods.

As used herein, "diagnosis" relates to the recognition and/or detection of a clinical condition, in the present case the presence of cancer in a patient. The patients of the present invention have been diagnosed as having a cancer. As explained above, it is understood that in the context of the present invention the "time point of diagnosis" is the time point at which the treating physician or medical personnel suspects the patient of having ovarian cancer. At this time point, the treating personnel decides on the next steps for confirming the diagnosis (surgery/biopsy isolation and histological analysis, therapeutic measures, etc.). This is the earliest time point of sample isolation in the context of the method of the present invention and corresponds to the time point, from which the patient is classified as "diagnosed" with ovarian cancer, although a histological proof of the (suspected and later on confirmed") diagnosis may not be available, yet.

The method of the invention is a method for the prognosis of disease progression, in particular cancer progression in a patient. The term "prognosis" relates to the prediction of an outcome or a specific risk for a cancer patient, such as the risk of the occurrence of a subsequent adverse event as described herein. This may also include an estimation of the chance of recovery or the chance of an adverse outcome for said patient.

The method described herein comprises the determination of a level of at least one biomarker, namely HGF, which correlates with the likelihood of an adverse event in the health of a patient diagnosed with ovarian cancer. This preferably means that the likelihood of the occurrence of an adverse event can be assessed on the comparison of the determined level of HGF to a reference level (such as a threshold or cut-off value and/or a population average), wherein the reference level may correspond the level of HGF in patients who have not and will not experience the respective adverse event.

Accordingly, the determination of HGF as described herein may be used to assess whether the patient has an elevated or decreased likelihood for an adverse event. A level of HGF that indicates an elevated likelihood of an adverse event may thus indicate a high likelihood that the patient will suffer of an adverse event after sample isolation.

In terms prognosis of future adverse event the method is particularly suited for a risk assessment or stratification. This means, that the method of the invention can discriminate high risk patients, who are more likely to face an adverse event in the future, or whose state will become more critical in the future, from low risk patients, whose health state is stable or even improving, so that it is not expected that they will suffer from a certain adverse event. Based on this assessment it is possible to take therapeutic/treatment decisions based on the method of the invention, which can therefore also be used as a method for therapy guidance, therapy stratification and/or therapy monitoring.

In the present invention, the terms "risk assessment" and "risk stratification" relate to the grouping of subjects into different risk groups according to their further prognosis. Risk assessment also relates to stratification for applying preventive and/or therapeutic measures. The term "therapy stratification" in particular relates to grouping or classifying patients into different groups, such as risk groups or therapy groups that receive certain differential therapeutic measures depending on their classification. The term "therapy stratification" also relates to grouping or classifying patients with infections or having symptoms of an infectious disease into a group that are not in need to receive certain therapeutic measures.

The methods of the invention may also be used for monitoring. "Monitoring" relates to keeping track of an already diagnosed disorder e.g. to analyze the progression of the disease or the influence of a particular treatment or therapy on the disease progression. The term "therapy monitoring" or "therapy control" in the context of the present invention refers to the monitoring and/or adjustment of a therapeutic treatment of the patient, for example by obtaining feedback on the efficacy of the therapy. As used herein, the term "therapy guidance" refers to application of certain therapies, therapeutic actions or medical interventions based on the value/level of HGF and potentially further biomarkers, such as CA-125 and/or clinical parameter and/or clinical scores, such as FIGO staging. This includes the adjustment of a therapy or the discontinuation of a therapy.

As used herein, the term "sample" is a biological sample that is obtained or isolated from the patient or subject. "Sample" as used herein may, e.g., refer to a sample of bodily fluid or tissue obtained for the purpose of diagnosis, prognosis, or evaluation of a subject of interest, such as a patient. Preferably herein, the sample is a sample of a bodily fluid, such as blood, serum, plasma, cerebrospinal fluid, urine, saliva, sputum, pleural effusions, cells, a cellular extract, a tissue sample, a tissue biopsy, a stool sample and the like. Particularly, the sample is blood, blood plasma, blood serum, or urine.

Embodiments of the present invention refer to the isolation more than one sample at different time points, such as at the time point of diagnosis and/or at one or more time points over the course of a treatment initiated upon diagnosis. Such samples may be referred to as a first sample and a second sample and so on. In the context of the method of the present invention, the terms "first sample" and "second sample" relate to the relative determination of the order of isolation of the samples employed in the method of the present invention. When the terms first sample and second sample are used in specifiying the present method, these samples are not to be considered as absolute determinations of the number of samples taken. Therefore, additional samples may be isolated from the patient before, during or after isolation of the first and/or the second sample, or between the first or second samples, wherein these additional samples may or may not be used in the method of the present invention. The first sample may therefore be considered as any previously obtained sample. The second sample may be considered as any further or subsequent sample.

Preferably, the one or more samples of the method of the invention are blood samples. The term "blood sample" comprises without limitation serum, plasma or whole blood samples.

"Plasma" in the context of the present invention is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (e.g. citrated, EDTA or heparinized blood), for example for at least 15 minutes at 2000 to 3000 g.

"Serum" in the context of the present invention is the liquid fraction of whole blood that is collected after the blood is allowed to clot. When coagulated blood (clotted blood) is centrifuged serum can be obtained as supernatant.

In one aspect, the invention also relates to a kit for carrying out the method for prognosing disease progression of the invention.

The kit of the invention comprises reference data, or means to obtain reference data, for the risk of a patient as to whether a subsequent adverse event in the health of said patient will occur.

As used herein, "reference data" comprise reference level(s) of HGF and optionally other markers or clinical scores as CA125 and/or FIGO grading. The levels of HGF in the sample of the subject can be compared to the reference levels comprised in the reference data of the kit. The reference levels are herein described above and are exemplified also in the appended examples. The reference data can also include a reference sample to which the level of HGF and optionally further markers is compared. The reference data can also include an instruction manual how to use the kits of the invention.

Such reference data preferably include or correspond to HGF severity levels corresponding to a risk threshold or cut-off value, wherein preferably said reference data is stored on a computer readable medium and/or employed in the form of computer executable code configured for comparing the determined levels of HGF with the reference data, such as the HGF severity levels, risk threshold or cut-off value.

The methods of the present invention may in part be computer-implemented. For example, the step of comparing the detected level of HGF or fragments thereof, with a reference level can be performed in a computer system. In the computer-system, the determined level of the marker(s) can be combined with other marker levels and/or parameters of the subject in order to calculate a score, which is indicative for the prognosis, risk assessment and/or risk stratification. For example, the determined values may be entered (either manually by a health professional or automatically from the device(s) in which the respective marker level(s) has/have been determined) into the computer-system. The computer-system can be directly at the point-of-care (e.g. primary care, ICU or ED) or it can be at a remote location connected via a computer network (e.g. via the internet, or specialized medical cloud-systems, optionally combinable with other IT-systems or platforms such as hospital information systems (HIS)). Typically, the computer-system will store the values (e.g. marker level or parameters such as age, blood pressure, weight, sex, etc. or clinical scoring systems such as FIGO etc.) on a computer-readable medium and calculate the score based-on pre-defined and/or pre-stored reference levels or reference values. The resulting score will be displayed and/or printed for the user (typically a health professional such as a physician). Alternatively, or in addition, the associated prognosis, assessment, treatment guidance, or stratification will be displayed and/or printed for the user (typically a health professional such as a physician).

### FIGURES

The invention is further described by the following figures. These are not intended to limit the scope of the invention but represent preferred embodiments of aspects of the invention provided for greater illustration of the invention described herein.

### Brief description of the figures:

**Figure 1****:** sHGF levels in the course of surgery and adjuvant platinum-based chemotherapy.
**Figure 2****:** Association of sHGF level with clinicopathological data of ovarian cancer.
**Figure 3****:** sHGF levels and its association with cytoreductive surgery and histologic subtype.
**Figure 4****:** Prognostic relevance of sHGF at primary diagnosis and in the course of platinum-based chemotherapy.
**Figure 5****:** Prognostic relevance of the patient individual HGF dynamics.
**Figure** 6: sHGF is an independent prognostic biomarker for patients with confirmed BRCA1/2 wild-type ovarian cancer.

### Detailed description of the figures:

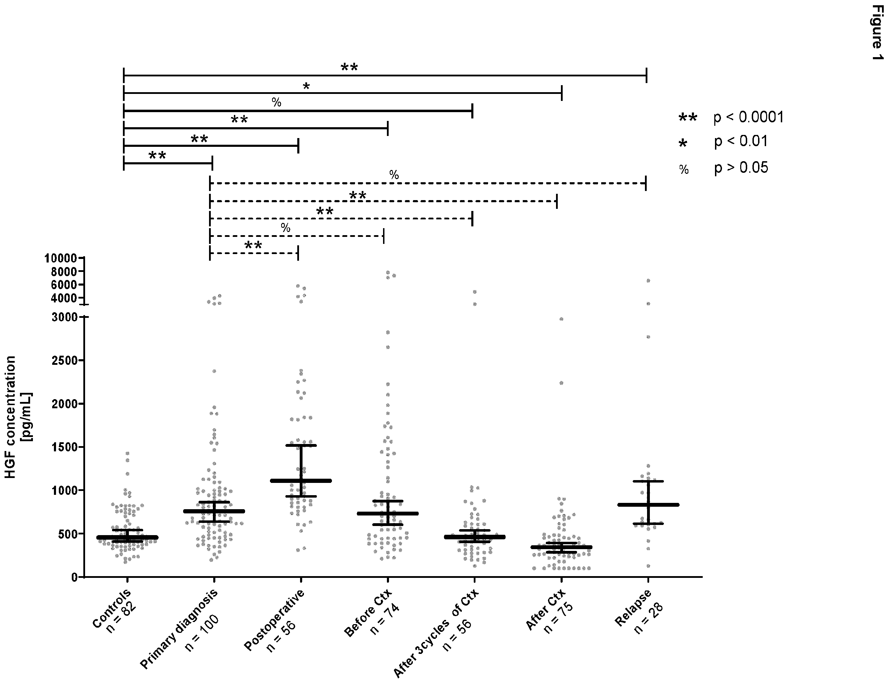

**Figure 1****:** *sHGF levels in the course of surgery and adjuvant platinum-based chemotherapy.* Scatter plot showing sHGF levels in healthy controls, in ovarian cancer patients at primary diagnosis and among five additional longitudinal follow-up samples, obtained (1) within one week after primary surgery (2) before platinum-based chemotherapy (3) after the third cycle of chemotherapy (4) after completion of chemotherapy and (5) at recurrence. The black horizontal lines indicate the median HGF levels in each group with error bars, showing the inter-quartile range. P-values, according to the two-tailed Mann-Whitney-U test for independent samples, are indicated.

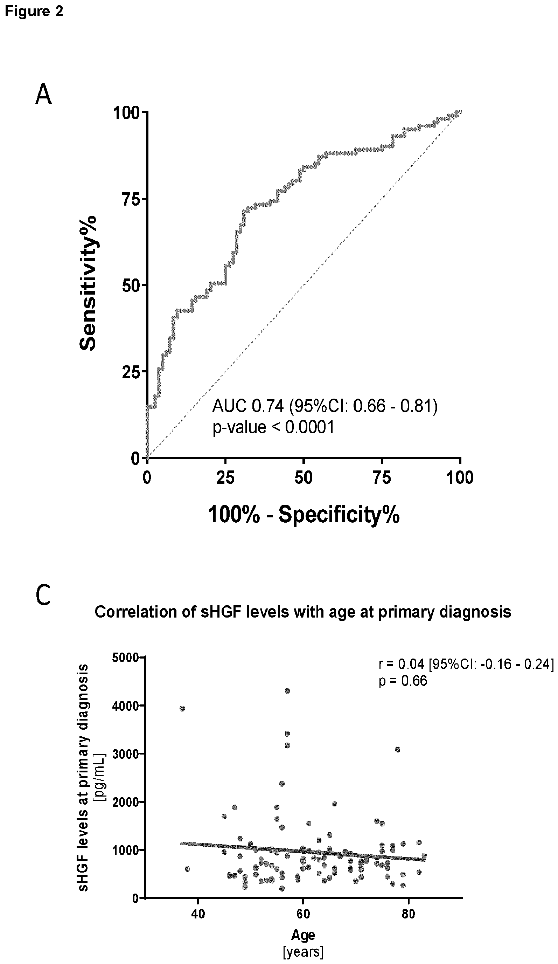

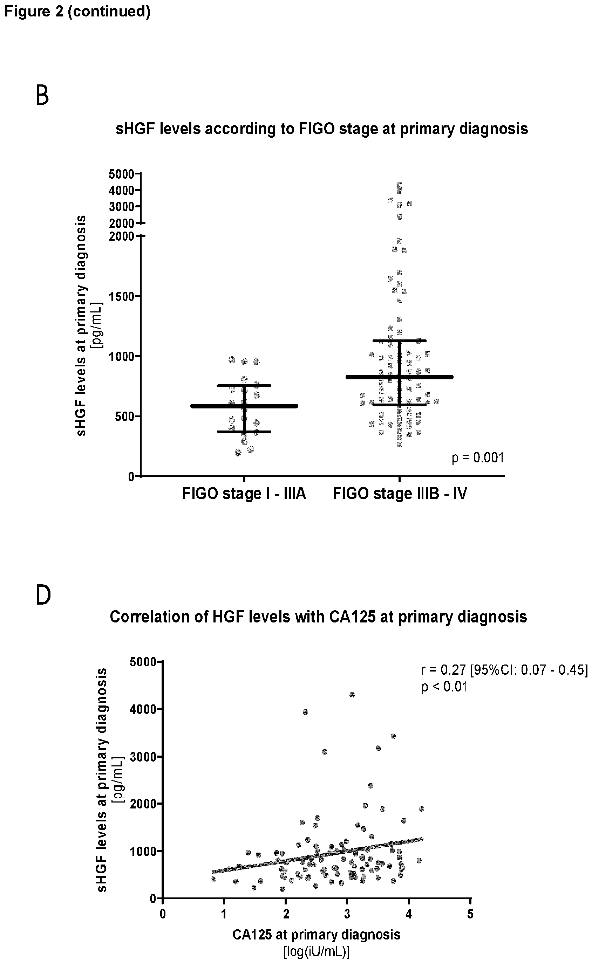

**Figure 2****:** *Association of sHGF level with clinicopathological data of ovarian cancer.* (**A**) Receiver operating characteristic (ROC) analysis to determine the diagnostic ability of serum HGF level to distinguish between ovarian cancer patients (total cohort) and healthy controls. The respective area under the curve (AUC) value and the 95% confidence interval (CI) are indicated. (**B**) Scatter plots comparing serum HGF level between FIGOI-IIIA vs. FIGO IIIB-IV ovarian cancer patients. The black horizontal lines indicate the median HGF levels in each group with error bars, showing the inter-quartile range. (**C+D**) Spearman correlation analysis of serum HGF at primary diagnosis and (C) the patients' age or (**D**) baseline CA125 values.

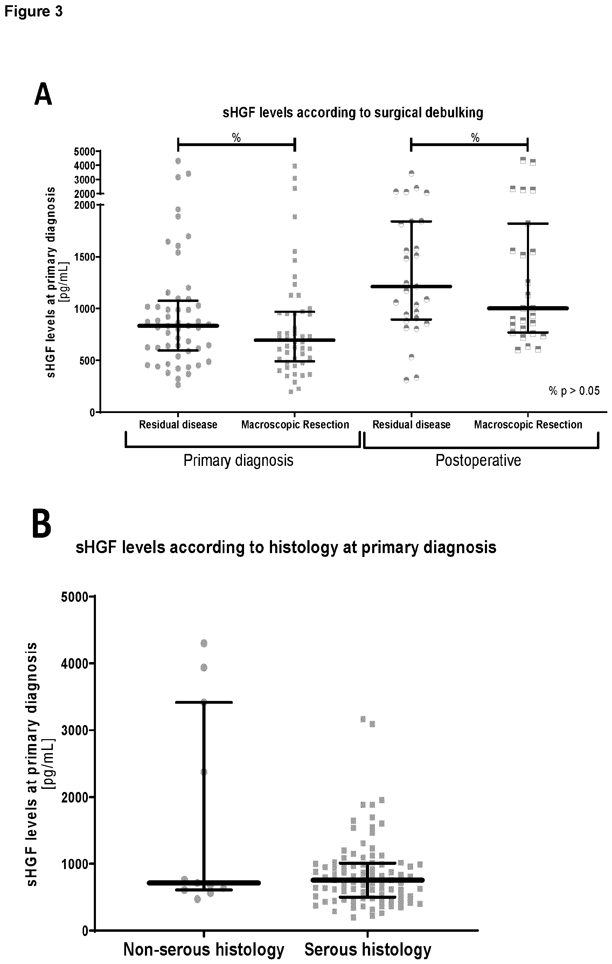

**Figure 3****:** *(**A**) Cytoreductive surgery by sHGF level.* Scatter plots comparing serum HGF level between patients with and without residual tumor left after primary debulking at primary diagnosis or within one week after surgery. *(**B**) sHGF according to histologic subtype at primary diagnosis.* Scatter plots comparing serum HGF level between non-serous and serous histologic subtypes. The black horizontal lines indicate the median sHGF levels in each group with error bars, showing the inter-quartile range.

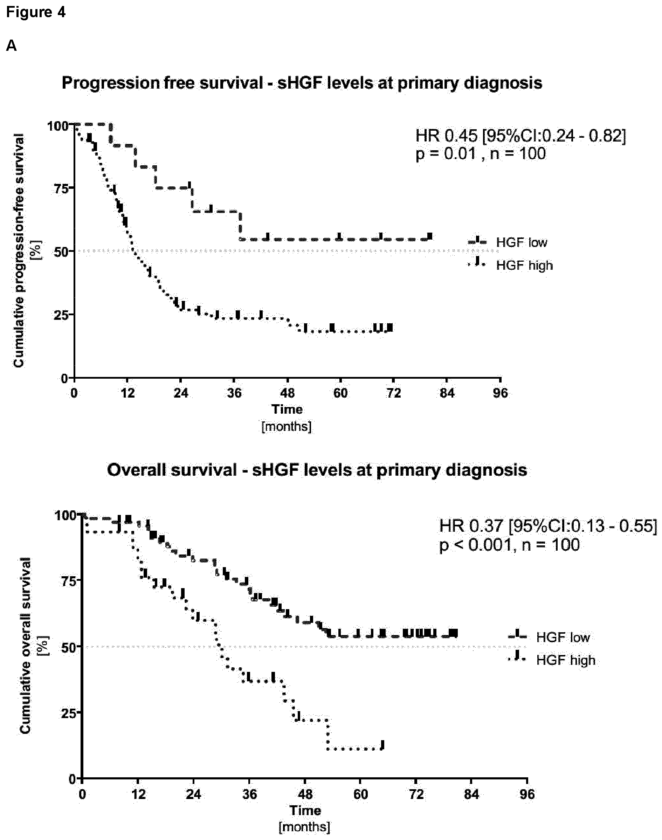

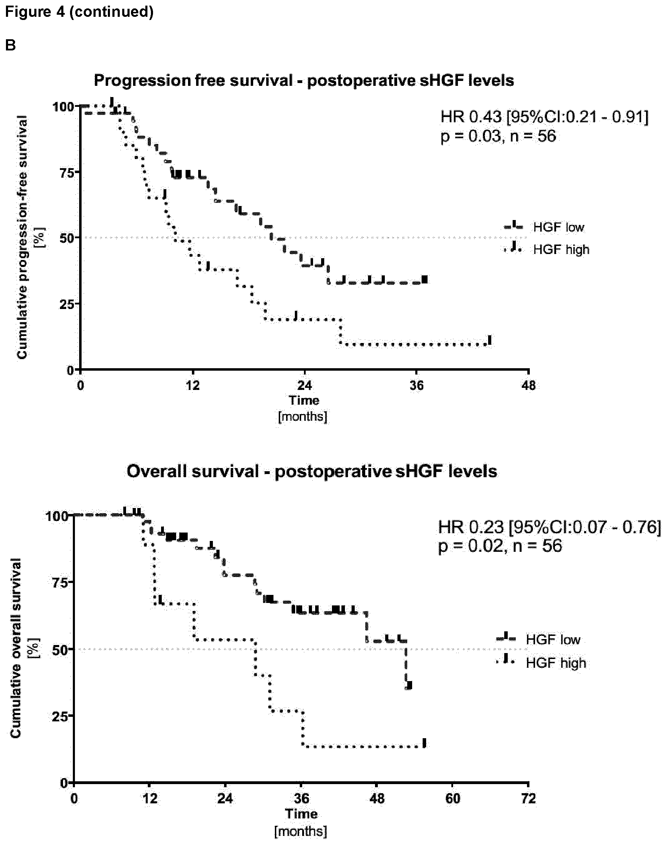

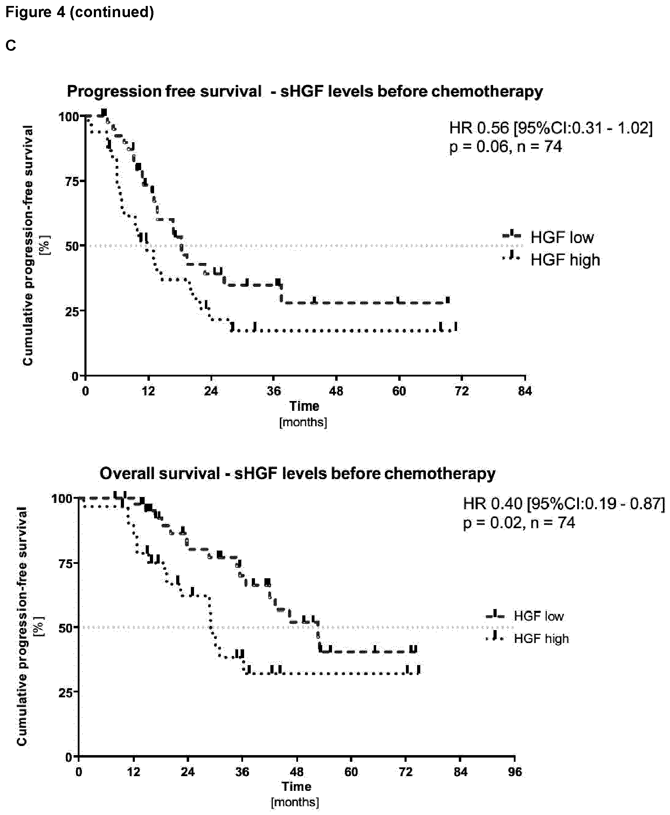

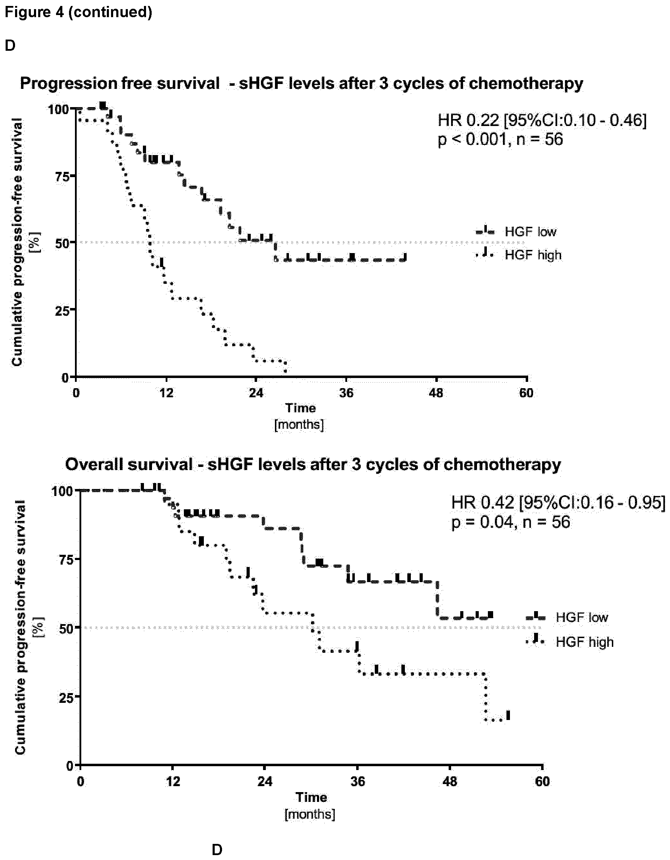

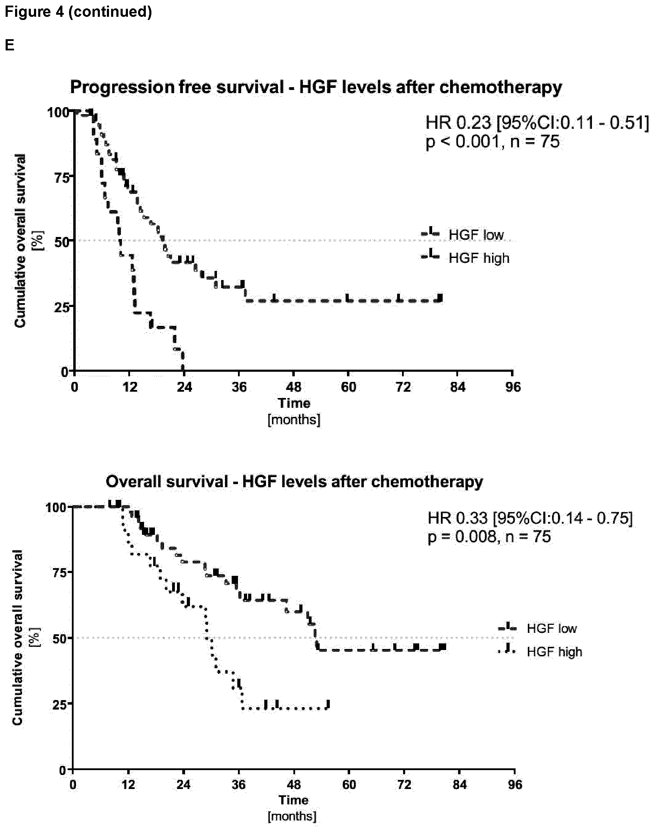

**Figure 4****:** *Prognostic relevance of sHGF at primary diagnosis and in the course of platinum-based chemotherapy.* Kaplan-Meier analysis comparing progression-free survival (PFS) and overall survival (OS) of patients with high sHGF level vs. patients with low serum HGF level (**A**) at primary diagnosis (**B**) after surgery (**C**) before the onset of platinum-based chemotherapy and (**D**) after three cycles of chemotherapy and (**E**) after chemotherapy.

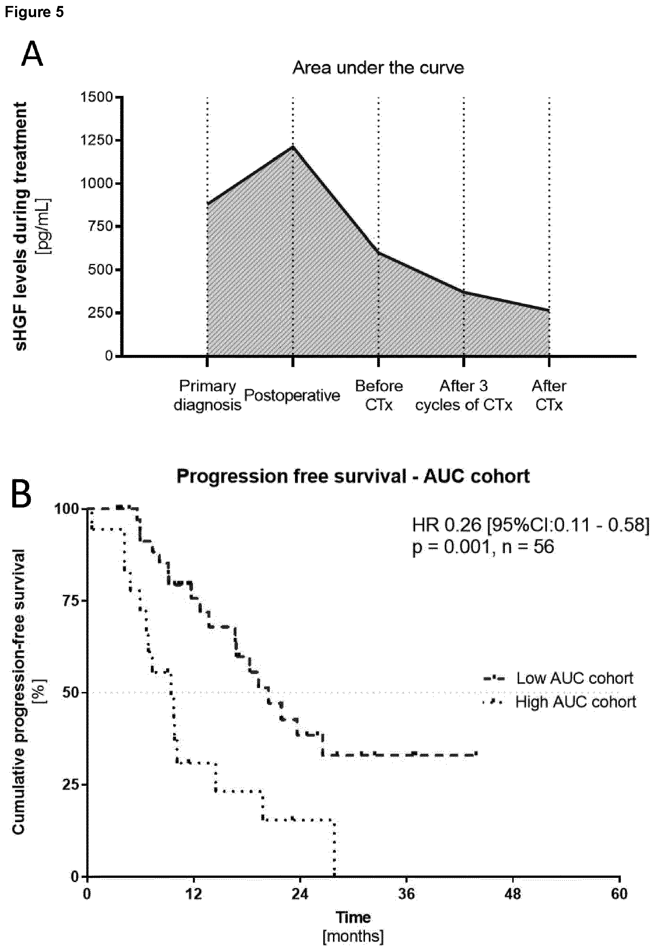

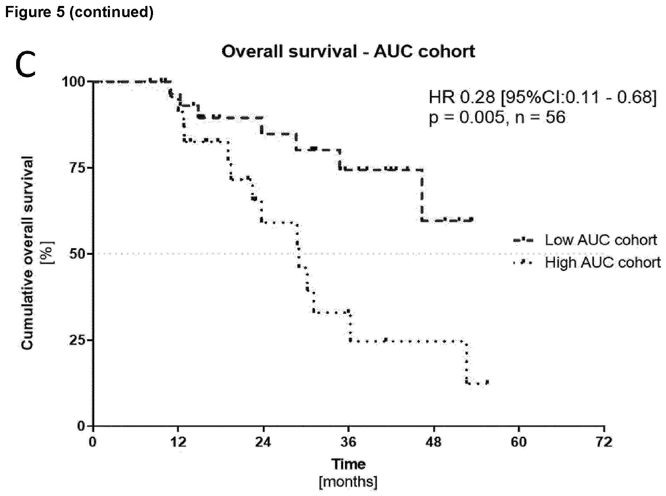

**Figure 5****:** *Patients' dynamic curves showing the progression of serum HGF levels in the time corridor between primary diagnosis and the completion of adjuvant chemotherapy.* (**A**) Example of a patient's AUC. (**B+C**) Kaplan-Meier analysis comparing (**B**) progression-free survival (PFS) and (**C**) overall survival (OS) of ovarian cancer patients with a high AUC value vs. patients with a low AUC value.

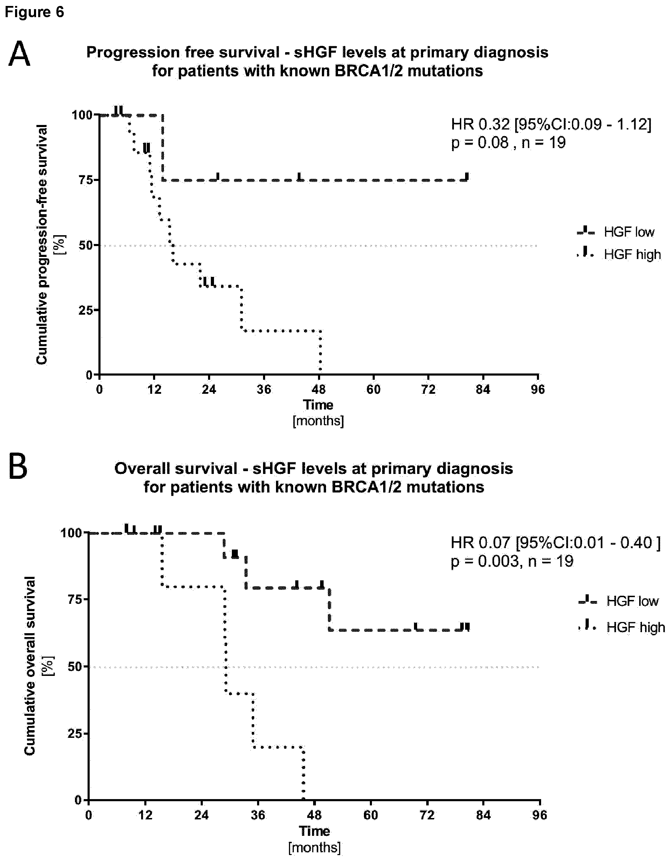

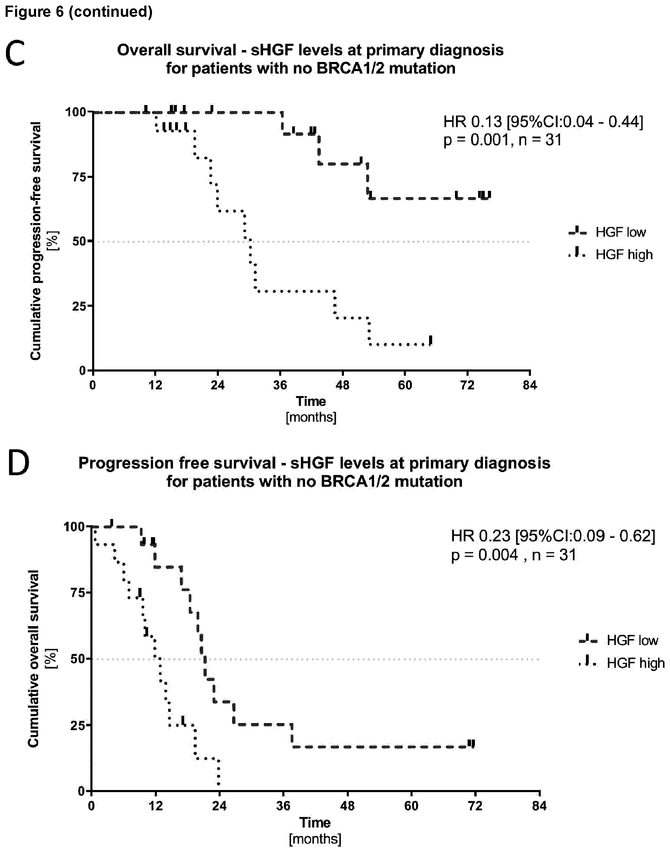

**Figure 6****:** *sHGF is an independent prognostic biomarker for patients with confirmed BRCA1*/*2 wild-type ovarian.* Kaplan-Meier analysis comparing progression-free survival (PFS) and overall survival (OS) of patients with high sHGF level vs. patients with low sHGF level at primary diagnosis in patients (**A, B**) with known BRCA1/2 mutant ovarian cancers (**C, D**) with known non-BRCA1/2-mutant ovarian cancer. BRCA1/2 mutant ovarian cancers include those patients with germline BRCA1/2 mutations or somatic BRCA1/2 mutations in cancerous tissue.

### EXAMPLES

The invention is further described by the following examples. These are not intended to limit the scope of the invention but represent preferred embodiments of aspects of the invention provided for greater illustration of the invention described herein.

### Patients and Methods of the Examples

### Patient characteristics

The present study was conducted at the Department of Gynecology and Obstetrics at the Carl Gustav Carus University of Dresden, Technische Universität Dresden, Dresden, Germany. In total, 113 patients with histologically confirmed primary epithelial ovarian cancer and adjuvant treatment were included. Informed written consent was obtained from all patients, and the Local Research Ethics Committee in Dresden has approved the study (EK74032013). The patients' clinical data are reported in **Table 2.** Tumors were classified in line with the WHO-classification of tumors derived from female genital tract and tumor staging was classified according to the Fédération Internationale de Gynécologie et d'Obstétrique (FIGO), which was revised in 2014 (30). The revised version of the FIGO-classification was used for all patients, who underwent primary surgery in 2014 or later. The whole study population patients received primary radical surgery aiming at macroscopic complete tumor resection followed by adjuvant platinum- and paclitaxel-based adjuvant chemotherapy. In case of no contraindications, patients with a tumor stage of at least FIGO IIIb were additionally treated with the monoclonal antibody bevacizumab.

**Table 2. Patient characteristics.**

| | |
|---|---|
| **N (total patient cohort)** | 113 |
| **Age** | median 60 years (35 - 82 years) |

| **BMI** | |
|---|---|
| recorded | 101 (89.4%) median 25.9 (17.1 - 50.3) |
| unknown* | 12 (10.6%) |

| **FIGO** | |
|---|---|
| I - II | 13 (11.5%) |
| III - IV | 100 (88.5%) |

| **Histologic type** | |
|---|---|
| serous | 101 (89.4%) |
| other | 12 (10.6%) |

| **Residual Tumor** | |
|---|---|
| macroscopic complete resection | 51 (45.1%) |
| any residual tumor | 62 (54.9%) |

| **BRCA status** | |
|---|---|
| unknown | 56 (49.6%) |
| BRCA1/2 mutation | 23 (20.4%) |
| No BRCA1/2 mutation | 34 (30.0%) |

| **Recurrence** | |
|---|---|
| PFS | median 13 months (1 - 140 months) |
| no Relapse | 35 (31.0%) |
| relapse | 78 (69.0%) |

| **Survival** | |
|---|---|
| OS | median 35 months (1 - 188 months) |
| alive | 61 (54.0%) |
| dead | 52 (46.0%) |

| | |
|---|---|
| *includes cases where samples were obtained only at disease recurrence and surgery performed >10 yrs ago and no recording was made available. | |

### Healthy controls

In total, 82 female healthy individuals without any history of benign or malignant disease were recruited for control serum samples. Informed written consent was obtained from all donors, and the Local Research Ethics Committee in Dresden has approved the study (EK74032013). There was no correlation between sHGF and age as confirmed by our own data set (r = 0.04, 95%CI: - 0.16 - 0.24, p = 0.66). Preparations of control samples and patient samples were performed with exactly the same protocol, in order to allow comparison.

### Serum preparation

After blood withdrawal with a 7.5 ml S-Monovette^{®} (Sarstedt AG & Co., Nuembrecht, Germany), blood samples were incubated at room temperature for at least 30 min to allow complete blood coagulation. Within 1h after blood drawing, serum was prepared by centrifugation for 8 min at 1,800 g at room temperature. The obtained cell free serum fraction was immediately frozen at -80 °C until further processing. Unnecessary freeze-thaw cycles were avoided. Samples were thawed on ice and were immediately processed after complete thawing. Samples were blinded so that neither time of blood drawing nor any other information could be disclosed during investigation.

### Detection of serum HGF

Serum derived HGF (sHGF) concentrations were determined with the commercially available enzyme-linked immunosorbent assay kit HGF ELISA kit (Thermo Fisher) and the assays were performed, according to manufacturer's instructions. The final optical readout was conducted with the microplate reader InfiniteM200 and software Magellan version 7.2 (Tecan, Männedorf, Switzerland).

### Statistical analysis

The statistical analysis was conducted with R, Version 3.6.2 and GraphPad Prism version 8.4.3 (GraphPad Software, La Jolla, CA, USA). P-values < 0.05 were considered statistically significant. All confidence intervals (CIs) were specified as 95%CI. Non-parametric two-sided Mann-Whitney-test was used to compare serum HGF levels. The Hodges-Lehman estimate was used to determine the estimated differences of medians. Uni- and multivariate Cox proportional hazard regression model analyses were performed to study the prognostic relevance of serum HGF in ovarian cancer and hazard ratios (HRs) are indicated with 95%CI. To predict platinum resistance, generalized linear model analysis was performed and odds ratio (OR). Additionally, Kaplan-Meier analyses were performed with significance levels indicated with log-rank (Mantel-Cox) analysis and and HRs (Mantel-Haenszel) are shown with 95%CI. All models were tested for OS and PFS as separate outcome variables. The correlation between serum HGF levels at primary diagnosis, age, CA125 and at recurrence was assessed by Spearman's rank-order test. For all regression and Kaplan-Meier analyses, the cutoff for stratifying the patients either into a HGF high or a HGF low group was defined using maximally selected rank statistics (**Table 3**). The median was used as cut off determination for stratifying the patients into area under the curve (AUC) high or AUC-low. Using receiver operating characteristic (ROC) curve analysis, we assessed the capacity of serum HGF concentrations to discriminate between ovarian cancer patients and healthy controls.

### Results of the Examples

### Median HGF level is highly elevated at primary diagnosis of ovarian cancer and is normalized by chemotherapy

We quantified sHGF levels in a comprehensive cohort of clinically documented ovarian cancer patients (n = 113) and compared it to levels in healthy controls (n = 82). Median sHGF level in baseline samples at primary diagnosis was highly elevated compared to healthy controls with an estimated difference (ED) of 241.8 (95%CI: 157.3 - 337.3, p < 0.0001, **Figure 1**). This was associated with the fact that HGF level allowed a good discriminative power between patients and control with an area under the curve (AUC) of 0.73 (95%CI: 0.66 - 0.80, p < 0.0001) in the total patient cohort (**Figure 2A**).

We determined sHGF level in the course of primary surgery and adjuvant chemotherapy, as represented by four longitudinal follow-up serum samples, which were available (1.) within one week after primary surgery (n=56), (2.) before the onset of platinum-based chemotherapy (n=74), (3.) after the first three cycles of chemotherapy (n=56), and (4.) after the completion of chemotherapy (n=75; **Figure 1**). After primary surgery, there was a strong increase of median HGF level compared to baseline levels at primary diagnosis with an ED of 393.4 (95%CI: 235.4 - 571.2, p < 0.0001), which again approached baseline levels before the onset of adjuvant chemotherapy. Already after the first three cycles of platinum-based chemotherapy, median sHGF level dropped down under the level of primary diagnosis, ultimately reaching the level of healthy controls after the completion of chemotherapy with an ED of -35.0 (95%CI: -111.2 to 45.2 , p = 0.4 after 3 cylces and dropped below the levels of healthy controls with an ED of -90.2 (95%CI: -147.9.8 to -30.9 , p = 0.005) after CTX. At the time point of first recurrence, median HGF-level recovered to that of primary diagnosis (**Figure 1**).

Conclusively, median sHGF level is elevated at primary diagnosis further transiently increases after surgery and responds already after three cycles of chemotherapy with a normalization to baseline levels to that of healthy controls.

### Association of HGF serum level with clinicopathological characteristics of ovarian cancer patients

Higher level of serum HGF reflected advanced disease, indicated by a FIGO-stage IIIB or higher (ED = 260.6, 95%CI: 101.8 - 448.3, p = 0.001; **Figure 2B**). There was no association between s HGF and the postoperative residual tumor burden left after primary debulking surgery, neither at the time of primary diagnosis nor after the completion of surgery (**Figure 3A**). HGF level at primary diagnosis was unrelated to the patient's age (r = 0.04, 95%CI: -0.16 - 0.24, p = 0.66); **Figure 2C**) and to the underlying histologic subtype (ED between non-serous vs. serous = -152.6 , 95%CI: - 1530 - 130.0, p = 0.29, **Figure 3B**). At primary diagnosis There was a weak correlation between sHGF and the of serum CA125 (log-values) (r = 0.27, 95%CI: 0.07 - 0.45 , p = 0.008; **Figure 2D**)**.** This would suggest that sHGF levels could also be used in combination with CA125 to offer further prognostic information for patients with ovarian cancer. In summary, sHGF levels does not correlate with age or histologic subtype. However, the combination of sHGF levels with further clinicopathological parameters such as FIGO stage and CA125 may offer additional benefit.

### Serum HGF at primary diagnosis is an independent predictor of survival

Univariate cox proportional hazards regression analysis with sHGF levels at primary diagnosis revealed that a higher sHGF level indicated reduced PFS (HR = 0.31, 95%CI: 0.13 - 0.79 0, p = 0.013) and reduced OS (HR = 0.35, 95%CI: 0.19 - 0.64, p < 0.001 ; **Table 4**). Kaplan-Meier survival analysis and the Log-Rank tests were additionally performed. Consistently, patients with a high sHGF level had shorter PFS (HR = 0.45, 95%CI: 0.24 - 0.82, p = 0.01) and shorter OS (HR = 0.37, 95%CI: 0.13 - 0.55, p < 0.001; **Figure 4A**).

We performed multivariate cox proportional hazards regression analysis with PFS or OS as outcome variables, including serum HGF levels and established risk factors of ovarian cancer, i.e. age, body mass index (BMI), residual tumor load left after primary debulking and FIGO-stage. We observed that an elevated HGF level was an independent predictor of shorter OS (HR = 0.41, 95%CI: 0.22 - 0.78, p = 0.006) but not PFS (HR = 0.40, 95%CI: 0.16 - 1.01, p = 0.058, **Table 5**).

### Serum HGF in the course of treatment is an independent predictor of survival

We analysed prognostic relevance of sHGF in the longitudinal follow-up samples, in an independent manner, by disregarding the individual time-dependent course of each patient. According to univariate cox regression analysis,a high level of sHGF at all investigated time points with the exception of the serum sample before the onset of platinum-based chemotherapy, indicated shorter PFS (after surgery: HR = 0.45 95%Cl: 0.23 - 0.90, p = 0.024; after the first three cycles of chemotherapy: HR = 0.26 95%Cl: 0.13 - 0.54 , p < 0.001; after the completion of chemotherapy: HR = 0.35 95%Cl: 0.19 - 0.64 , p < 0.001 **Table 4**). Moreover, high level of sHGF at all investigated time points were associated with shorter OS without any exception (after surgery: HR = 0.34, 95%Cl: 0.14 - 0.85, p = 0.021; before chemotherapy: HR = 0.44, 95%CI: 0.22 - 0.89, p = 0.023; after the first three cycles of chemotherapy: HR = 0.41, 95%CI: 0.17 - 0.98, p = 0.045; after the completion of chemotherapy: HR = 0.39 95%Cl: 0.19 - 0.80, p < 0.010, **Table 4**). The same results were obtained by Kaplan-Meier analysis and the logrank test (**Figure 4B-E**).

Taking into account other existing risk factors of ovarian cancer, multivariate cox regression analysis revealed that a higher level of sHGF at all in investigated time points is an independent predictor for either shorter PFS (after three cycles of chemotherapy: HR = 0.37, 95%CI: 0.17 - 0.20:, p = 0.012), shorter OS (before chemotherapy: HR = 0.44, 95%CI: 0.44 - 0.94, p = 0.035) or for both, shorter PFS and OS, together (PFS after surgery HR = 0.29, 95%CI: 0.14 - 0.63, p = 0.001; OS after surgery HR = 0.09, 95%CI: 0.02 - 0.36, p <0.001; PFS after chemotherapy HR = 0.48, 95%CI: 0.25 - 0.92, p = 0.027; OS after chemotherapy HR = 0.43, 95%Cl: 0.20 - 0.91, p = 0.019, **Table 5**).

To sum up, we demonstrate that the individual progression of sHGF levels is an accurate and independent prognostic biomarker looking at individual time points along the entire primary treatment.

### The patient individual dynamic of serum HGF is an independent predictor of survival

From 56/113 patients, a complete set of serum samples at primary diagnosis and in matched longitudinal serum samples was available. We inquired the prognostic relevance of sHGF level in each individual patient. Assuming a linear and continuous change of sHGF levels between the investigated time points, we plotted for each patient a dynamic curve using along primary surgery and adjuvant platinum-based chemotherapy. By setting the different time points of blood analysis as categorical variables, we calculated patient individual AUCs, each of them mirroring the individual sHGF dynamic in the course of primary treatment. For this purpose, patients were stratified into a "AUC high" group or into a "AUC low" group (**Figure 5A****+B**). The individual progression of serum HGF was prognostically significant and patients with a high AUC value had shorter PFS and OS, as indicated by univariate cox proportional hazards regression analysis (PFS: HR = 0.34 95%CI: 0.17 - 0.68, p = 0.002; OS: HR = 0.22, 95%Cl: 0.09 - 0.55, p = 0.001) and Kaplan-Meier analysis (PFS: HR = 0.26 95%CI: 0.11 - 0.58, p = 0.001; OS: HR = 0.28 95%CI: 0.11 - 0.68, p = 0.005). Multivariate analysis confirmed that this was independent from established risk factors of ovarian cancer (PFS: HR = 0.24, 95%CI: 0.11 - 0.53, p < 0.001; OS: HR = 0.21, 95%CI: 0.07 - 0.63, p = 0.005).

Taken together, we report that individual progression of sHGF is an independent prognostic biomarker for ovarian cancer patients.

### Serum HGF level and BRCA1/2 mutational status

The use of PARP inhibitors has already shown to drastically improve PFS in patients with BRCA1/2 mutant ovarian cancer and it will most likely also improve overall survival for this selected patient cohort. Since the use of PARP inhibitors in the first-line has until recently been restricted to comparatively few patients with BRCA1/2 mutant ovarian cancers (about 20%), it is of pivotal clinical importance to stratify patients with BRCA1/2 wild-type tumors (about 80% of all patients with ovarian cancer). The clinical response (PFS) has not been as dramatic in this patient cohort (6, 7), which is the reason why patients with non-BRCA1/2 mutant ovarian cancer will most likely benefit from further stratification. By evaluating patients who had gBRCA1/2 mutations ruled out by genetic testing, we were able to show that measuring sHGF levels gives prognostic information in this patient cohort (wtBRCA1/2) at primary diagnosis (**Table 6 and** **Figure 6**). The confirmed wtBRCA1/2 patients with high a HGF level at primary diagnosis showed reduced PFS (HR 0.21, 95%CI: 0.06 - 0.78 , p < 0.020) and OS (HR 0.42 , 95%CI: 0.03 - 0.91 , p < 0.040) in the multivariate analysis (**Table 6**). These results were confirmed by Kaplan-Meier analysis (**Figure 6C****,D**). As expected, only a limited number of patients had confirmed BRCA1/2 mutations (n = 19). Nonetheless, a high sHGF level also indicated poor OS in patients with BRCA1/2 mutations at primary diagnosis (HR 0.07, 95%l: 0.01 - 0.40, p = 0.003, **Figure 6B**).

### Discussion of the examples and conclusions

We investigated clinical relevance of sHGF as potential blood-based biomarker for ovarian cancer patients. We reported that sHGF is elevated at primary diagnosis and at recurrence of ovarian cancer, compared to healthy controls, and that a higher HGF level at primary diagnosis and in the course of the disease is an independent predictor for shorter PFS and/or OS.

In vitro experiments have shown that the HGF/cMET pathway induces invasion and migration of ovarian cancer cells (30). Overexpression of cMET has been observed in up to 75% of ovarian cancer patients and was associated with poor prognosis (22,31). Moreover, HGF is present at high concentrations in malignant ascites of ovarian cancer patients and induces migration of human peritoneal mesothelial cells by activation of cMET (33). Regardless of cMET overexpression, which is frequently observed in ovarian cancer patients (22), cMET can be further stimulated by stromal derived HGF (34). Moreover, since ovarian cancer cell lines were shown to express activated phosphorylated cMET at Tyr1349 only in response to added HGF (34), it was proposed that cMET activation in cancer cells is dependent on ligand induced receptor dimerization and therefore occurs mostly in an HGF-dependent manner (35).

We compared serum HGF level to healthy controls and also report that HGF is highly elevated at primary diagnosis of ovarian cancer and at the time of recurrence, which allowed reasonable discrimination between ovarian cancer patients and healthy controls (AUC=0.71). Therefore, a statistically substantiated conclusion on the diagnostic capacity of serum HGF for blood-based detecting of low stage ovarian cancer was not possible.

The exact origin of sHGF and especially the mechanisms, underlying increased HGF levels in ovarian cancer patients have incompletely been understood and could be explained by two different scenarios. First of all, HGF-dependent c-MET activation in ovarian tumors could be exerted by an autocrine loop (36), by which HGF is expressed and supplied by the tumor cells themselves. In this scenario, the tumor itself may contribute to elevated HGF level in the blood, especially since HGF is present in malignant ascites (37) and HGF expression of ovarian cancer cells was shown to increase with the tumor stage (32). Considering that HGF is serologically dispersed all over the body (38), c-MET activation of tumor cells could be regulated by paracrine mechanisms, so that the cancer is dependent on environmental HGF from local HGF-producing sources, such as tumor-associated fibroblasts at the invasive front within the tumor stroma (34), or from other remote sources all over the body. Determining the origin of serum HGF in ovarian cancer patients is further complicated by the fact that elevated HGF levels are not necessarily indicative for cancer, as they were also observed in graft-versus-host or infections disease (39,40). Independently from the possible origin of serum HGF, our data suggest that serum HGF reflects the tumor load, since it correlated with FIGO stage and HGF level tightly approached to the level of healthy controls already after the first three cycles of platinum-based chemotherapy. In addition to the elevated serum HGF levels at primary diagnosis, we observed a strong and transient HGF increase after primary surgery. It is likely that physical traumata, conferred by surgery, may alter the rate of HGF release into circulation. This is in line with the fact that HGF level were shown to be transiently elevated following thoracic surgery (41). Moreover, HGF and cMET are upregulated after tissue damage, such as kidney, liver or heart injury, suggesting that the HGF/cMET pathway is involved in general mechanism of tissue damage response and tissue regeneration (42-44).

To the best of our knowledge, our study is the first that shows systematic investigation of longitudinal serum HGF levels of ovarian cancer patients in the course of primary treatment and at recurrence. As our key finding, we described that serum HGF level at primary diagnosis as well as in the course of the disease is an independent predictor of poor survival. This prognostic relevance sustained, regardless whether we considered the individual progression of HGF level or an independent analysis of the different points of therapy. Therefore, increased HGF level may be suitable for identifying patients with a more aggressive disease, i.e. those with a high risk of recurrence and higher mortality. Our study offers new insights into the potential clinical use of serum HGF as a discrete blood-based biomarker, which may be used in combination with CA-125 and could repeatedly be applied for prognostic stratification in terms of therapy monitoring. Furthermore, determining serum HGF level at primary diagnosis could be further investigated as a pharmacodynamics biomarker for predicting response to pharmacological approaches, targeting the HGF/cMET signalling axis (26,27,45).

There is a clinical need to predict whether or not a patient will be resistant to standard platinum-based chemotherapy. i.e. a disease relapse within the first 6 months after completion of chemotherapy. This is indicative of poor prognosis and a test to identify those patients prior to treatment would allow for a personalised and potentially more targeted therapeutic strategy. This would also avoid the side effects seen by platinum- and paclitaxel-based first line therapy. Measuring HGF levels prior to treatment offers such a predictive test. We were able to show that there is a trend that HGF levels at primary diagnosis can predict platinum resistance with an odds ratio (OR) of 2.643 in the univariate analysis and (OR) of 2.203 in the multivariate analysis (**Table 7**). In conclusion, a high sHGF level might be predictive for primary platinum resistance and it could offer further prognostic information for patients with ovarian cancer.

To the best of our knowledge, this is the first study, suggesting sHGF as a blood-based and independent prognostic biomarker for ovarian cancer patients at primary diagnosis and in the course of platinum-based chemotherapy. As a robust and easily detectable serum parameter, HGF could possibly be implemented into routine diagnostics as a CA-125 auxiliary tumor marker for individualized prognosis stratification in ovarian cancer and for monitoring treatment response. Patients with high risk of recurrence, as identified by increased sHGF, might benefit from intensified therapy regimens, such as PARP inhibitors or immunotherapy.

### Tables of the examples

**Table 3. List of sHGF cut off values. Determination of fixed sHGF level cut-offs by maximally selected logrank statistics for univariate, multivariate and Kaplan-Meier analysis, categorizing patients into sHGF high and sHGF low group with HR. (CTx =Chemotherapy).**

| **Cut off identification, using maximally selected LogRank statistics** | | | | |
|---|---|---|---|---|
| | **Progression free survival** sHGF levels [pg/mL] | | **Overall survival** sHGF levels [pg/mL] | |
| | **cut off** | **p-value** | **cut off** | **p-value** |
| **Primary diagnosis** | 401.00 | 0.019 | 970.42 | 0.022 |
| **After primary surgery** | 1516.42 | 0.206 | 2122.39 | 0.222 |
| **Before chemotherapy** | 826.67 | 0.445 | 860.58 | 0.256 |
| **After 3 cycles of chemotherapy** | 487.71 | 0.003 | 537.19 | 0.306 |
| **After chemotherapy** | 603.88 | 0.025 | 489.55 | 0.145 |
| **AUC cohort** | 4266.00 | 0.043 | 4261.00 | 0.008 |
| **mBRCA cohort** | 347.62 | 0.279 | 954.00 | 0.072 |
| **wtBRCA cohort** | 716.00 | 0.086 | 728.20 | 0.013 |
| **Platinum resistance cut off at primary diagnosis:** cut off = 1550 pg/mL, p-value = 0.356 | | | | |
| **Platinum resistance cut off after completion of CTx:** cut off = 456 pg/mL, p-value = 0.078 | | | | |

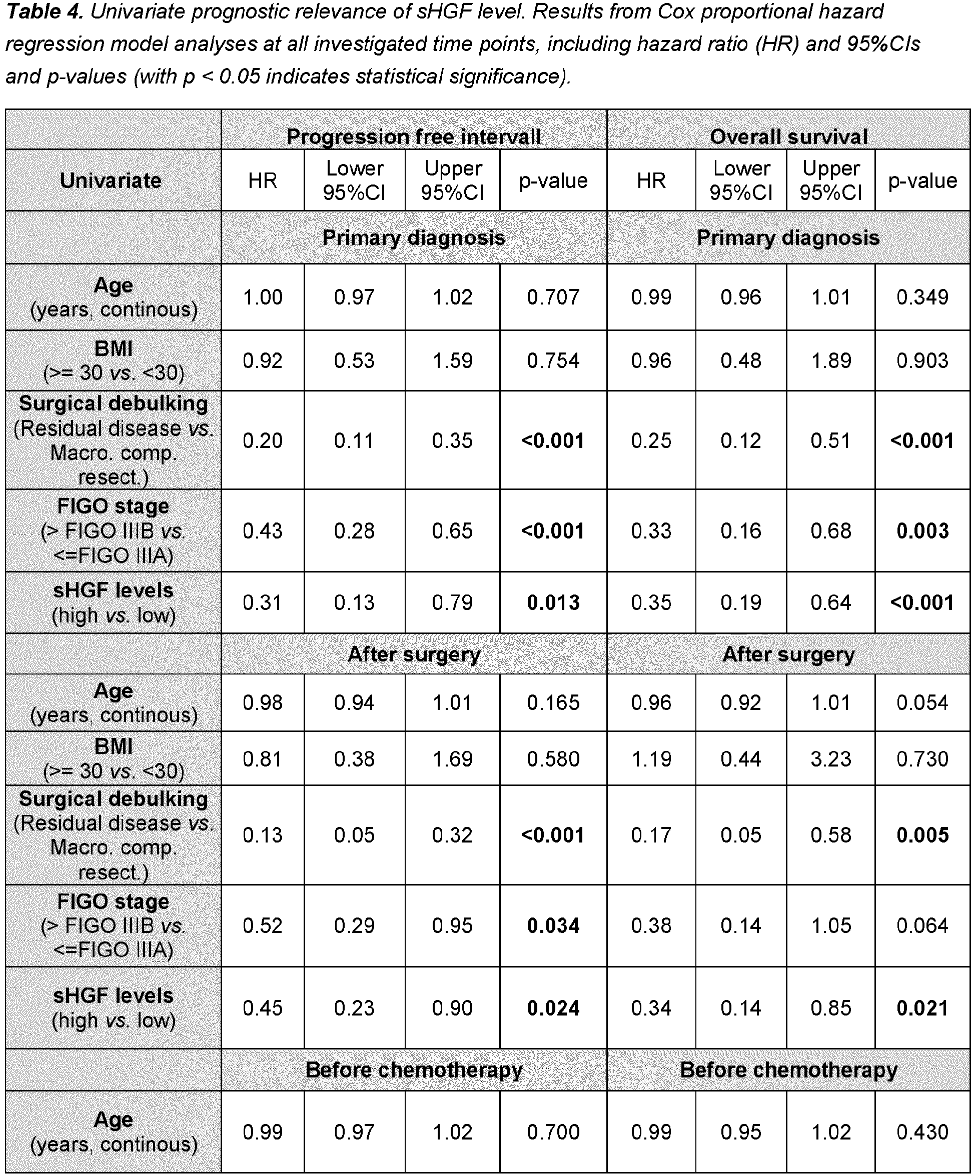

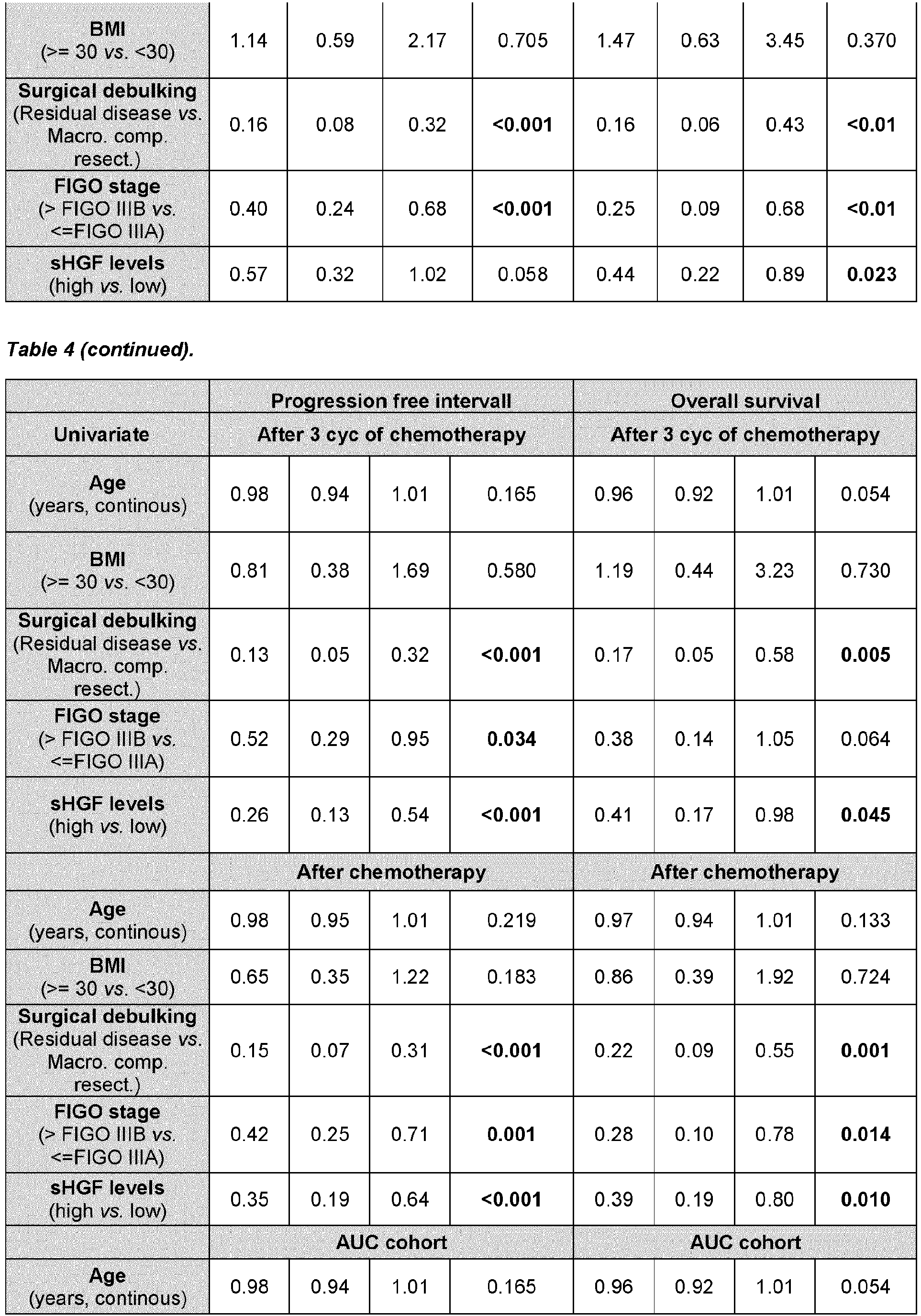

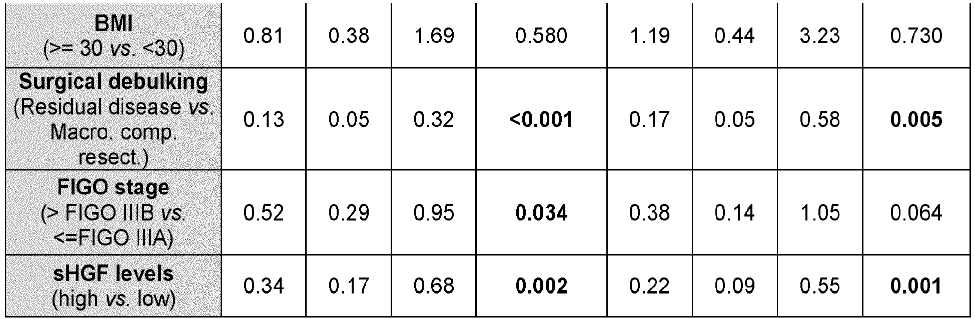

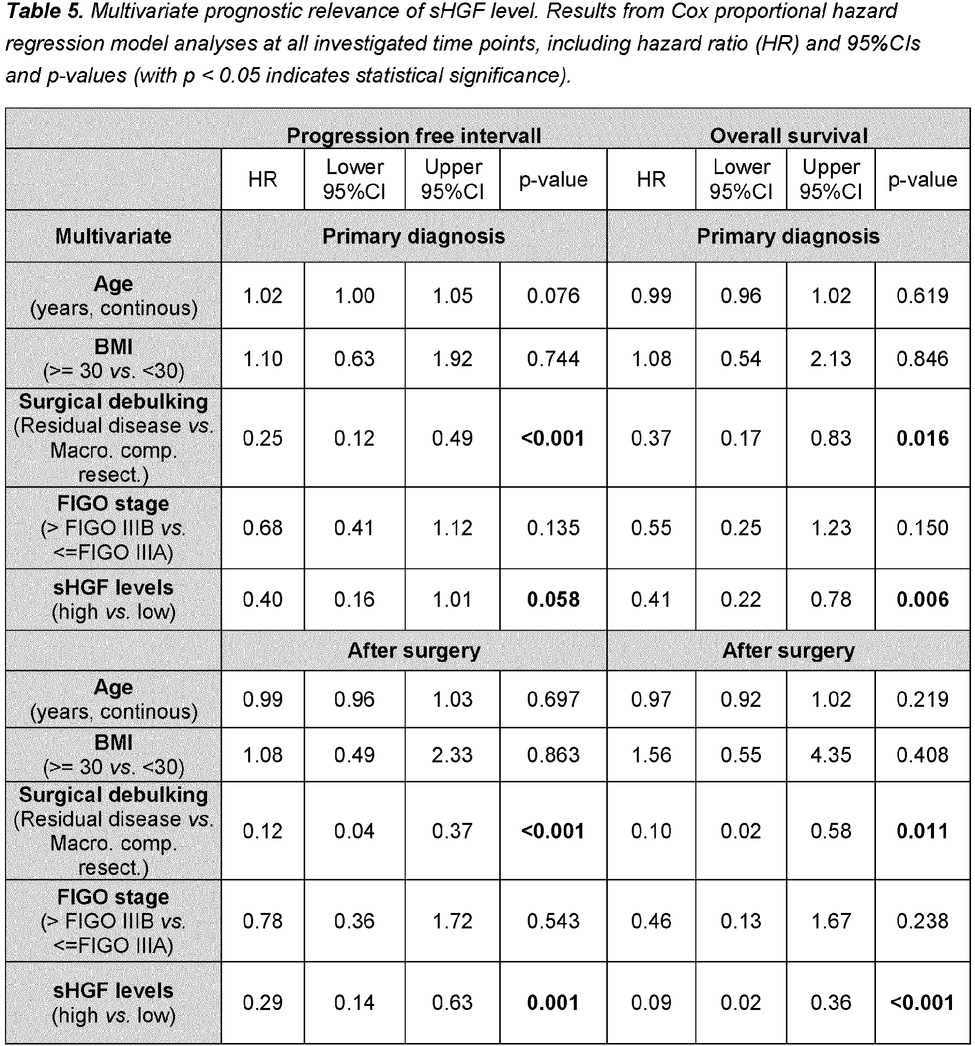

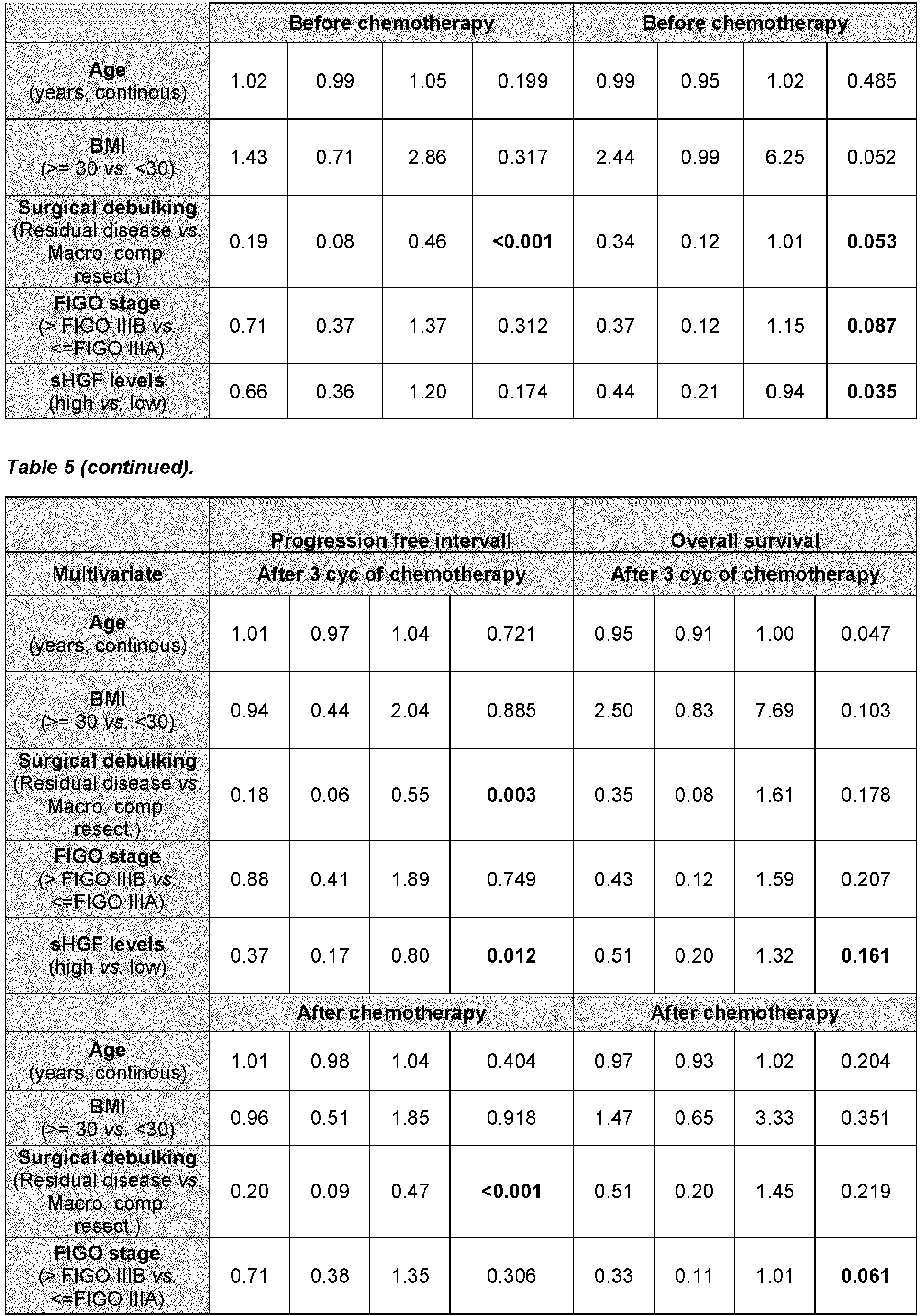

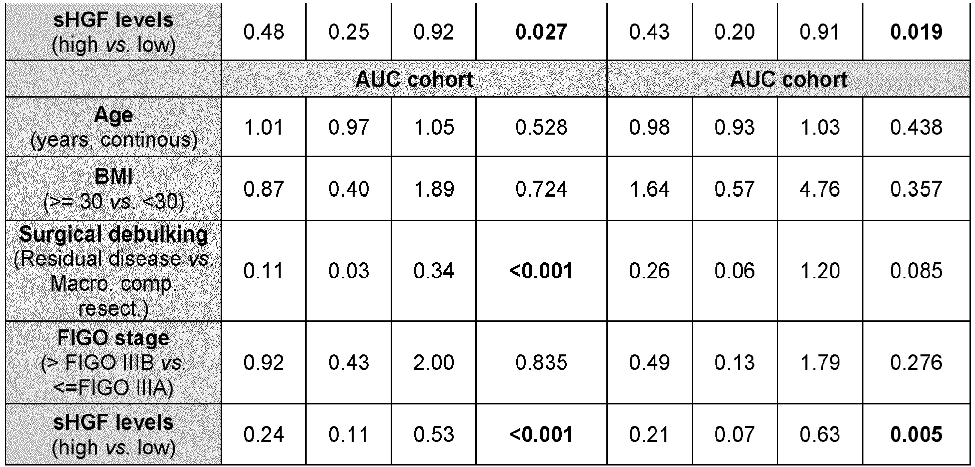

**Table 6. sHGF is an independent prognostic biomarker for patients with confirmed BRCA1/2 wild-type ovarian cancer. Multivariate analysis (PFS, OS) of sHGF levels in patients with confirmed non-BRCA1/2-mutant ovarian cancer.**

| | **Progression free survival** | | | | **Overall survival** | | | |
|---|---|---|---|---|---|---|---|---|
| | HR | Lower 95%CI | Upper 95%CI | p-value | HR | Lower 95%CI | Upper 95%CI | p-value |
| | **Primary diagnosis - no BRCA1/2 mutation** | | | | **Primary diagnosis - no BRCA1/2 mutation** | | | |
| **Age** (continous) | 1.02 | 1.00 | 1.05 | 0.067 | 0.93 | 0.85 | 1.02 | 0.125 |
| **BMI** (>= 30 *vs.* <30) | 0.74 | 0.22 | 2.50 | 0.630 | 0.73 | 0.10 | 5.56 | 0.760 |
| **Surgical debulking** (Residual disease vs. Macro. comp. resect.) | 0.48 | 0.12 | 1.89 | 0.294 | 1.75 | 0.23 | 14.29 | 0.588 |
| **FIGO stage** (> FIGO IIIB *vs.* <=FIGO IIIA) | 0.54 | 0.15 | 1.96 | 0.353 | 0.46 | 0.10 | 2.13 | 0.323 |
| **sHGF levels** (high *vs.* low) | 0.21 | 0.06 | 0.78 | **0.020** | 0.42 | 0.03 | 0.91 | **0.039** |

**Table 7. Prediction of platinum resistance with sHGF levels at primary diagnosis. Measuring sHGF level at primary can predict primary platinum resistance. Odds ratio were determined using generalised linear model was performed with univariate and multivariate values shown.**

| **Prediction of platinum resistance according to HGF levels at primary diaqnosis** | | |
|---|---|---|
| | **Univariate analysis OR** | **Multivariate analysis OR** |
| **Age** (years, continous) | 0.989 | 0.980 |
| **BMI** (>= 30 *vs*. <30) | 1.525 | 1.452 |
| **Surgical debulking** (Residual disease *vs*. Macro. comp. resect.) | 3.003 | 3.136 |
| **FIGO stage** (I, II. III, IV) | 1.325 | 1.009 |
| **sHGF levels** (high *vs*. low) (cut off = 1550 pg/mL) | 2.643 | 2.203 |

### References

1. Torre, L.A. et al. Ovarian cancer statistics, 2018. CA Cancer J Clin 68, 284-296 (2018).
2. du Bois, A. et al. 2004 consensus statements on the management of ovarian cancer: final document of the 3rd International Gynecologic Cancer Intergroup Ovarian Cancer Consensus Conference (GCIG OCCC 2004). Ann Oncol 16 Suppl 8, viii7-viii12 (2005).
3. Karam, A. et al. Fifth Ovarian Cancer Consensus Conference of the Gynecologic Cancer InterGroup: first-line interventions. Ann Oncol 28, 711-717 (2017).
4. Stuart, G.C. et al. 2010 Gynecologic Cancer InterGroup (GCIG) consensus statement on clinical trials in ovarian cancer: report from the Fourth Ovarian Cancer Consensus Conference. Int J Gynecol Cancer 21, 750-5 (2011).
5. Wimberger, P. et al. Influence of residual tumor on outcome in ovarian cancer patients with FIGO stage IV disease: an exploratory analysis of the AGO-OVAR (Arbeitsgemeinschaft Gynaekologische Onkologie Ovarian Cancer Study Group). Ann Surg Oncol 17, 1642-8 (2010).
6. Gonzalez-Martin, A. et al. Niraparib in Patients with Newly Diagnosed Advanced Ovarian Cancer. N Engl J Med 381, 2391-2402 (2019).
7. Ray-Coquard, I. et al. Olaparib plus Bevacizumab as First-Line Maintenance in Ovarian Cancer. N Engl J Med 381, 2416-2428 (2019).
8. Moore, K. et al. Maintenance Olaparib in Patients with Newly Diagnosed Advanced Ovarian Cancer. N Engl J Med 379, 2495-2505 (2018).
9. Nakamura, T., Nawa, K. & Ichihara, A. Partial purification and characterization of hepatocyte growth factor from serum of hepatectomized rats. Biochem Biophys Res Commun 122, 1450-9 (1984).
10. Cooper, C.S. et al. Molecular cloning of a new transforming gene from a chemically transformed human cell line. Nature 311, 29-33 (1984).
11. Bottaro, D.P. et al. Identification of the hepatocyte growth factor receptor as the c-met proto-oncogene product. Science 251, 802-4 (1991).
12. Landgraf, K.E. et al. An allosteric switch for pro-HGF/Met signaling using zymogen activator peptides. Nat Chem Biol 10, 567-73 (2014).
13. Furge, K.A., Zhang, Y.W. & Vande Woude, G.F. Met receptor tyrosine kinase: enhanced signaling through adapter proteins. Oncogene 19, 5582-9 (2000).
14. Garajova, I., Giovannetti, E., Biasco, G. & Peters, G.J. c-Met as a Target for Personalized Therapy. Transl Oncogenomics 7, 13-31 (2015).
15. Guglielmo, M.C. et al. The effect of hepatocyte growth factor on the initial stages of mouse follicle development. J Cell Physiol 226, 520-9 (2011).
16. Parrott, J.A. et al. Stromal-epithelial interactions in the progression of ovarian cancer: influence and source of tumor stromal cells. Mol Cell Endocrinol 175, 29-39 (2001).
17. Parrott, J.A., Vigne, J.L., Chu, B.Z. & Skinner, M.K. Mesenchymal-epithelial interactions in the ovarian follicle involve keratinocyte and hepatocyte growth factor production by thecal cells and their action on granulosa cells. Endocrinology 135, 569-75 (1994).
18. Birchmeier, C., Birchmeier, W., Gherardi, E. & Vande Woude, G.F. Met, metastasis, motility and more. Nat Rev Mol Cell Biol 4, 915-25 (2003).
19. Lorenzato, A. et al. Novel somatic mutations of the MET oncogene in human carcinoma metastases activating cell motility and invasion. Cancer Res 62, 7025-30 (2002).
20. Mhawech-Fauceglia, P., Afkhami, M. & Pejovic, T. MET/HGF Signaling Pathway in Ovarian Carcinoma: Clinical Implications and Future Direction. Patholog Res Int 2012, 960327 (2012).
21. Lee, J.H. et al. A novel germ line juxtamembrane Met mutation in human gastric cancer. Oncogene 19, 4947-53 (2000).
22. Kim, J.H., Jang, H.J., Kim, H.S., Kim, B.J. & Park, S.H. Prognostic impact of high c-Met expression in ovarian cancer: a meta-analysis. J Cancer 9, 3427-3434 (2018).
23. Sawada, K. et al. c-Met overexpression is a prognostic factor in ovarian cancer and an effective target for inhibition of peritoneal dissemination and invasion. Cancer Res 67, 1670-9 (2007).
24. Ayhan, A., Ertunc, D., Tok, E.C. & Ayhan, A. Expression of the c-Met in advanced epithelial ovarian cancer and its prognostic significance. Int J Gynecol Cancer 15, 618-23 (2005).
25. Zhou, H.Y. & Wong, A.S. Activation of p70S6K induces expression of matrix metalloproteinase 9 associated with hepatocyte growth factor-mediated invasion in human ovarian cancer cells. Endocrinology 147, 2557-66 (2006).
26. Vergote, I.B. et al. A phase 2 randomised discontinuation trial of cabozantinib in patients with ovarian carcinoma. Eur J Cancer 83, 229-236 (2017).
27. Martin, L.P. et al. A phase II evaluation of AMG 102 (rilotumumab) in the treatment of persistent or recurrent epithelial ovarian, fallopian tube or primary peritoneal carcinoma: a Gynecologic Oncology Group study. Gynecol Oncol 132, 526-30 (2014).
28. Current FIGO staging for cancer of the vagina, fallopian tube, ovary, and gestational trophoblastic neoplasia. Int J Gynaecol Obstet 105, 3-4 (2009).
29. Prat, J. & Oncology, F.C.o.G. Staging classification for cancer of the ovary, fallopian tube, and peritoneum. Int J Gynaecol Obstet 124, 1-5 (2014).
30. Ueoka, Y. et al. Hepatocyte growth factor modulates motility and invasiveness of ovarian carcinomas via Ras-mediated pathway. Br J Cancer 82, 891-9 (2000).
31. Liu, S. et al. Toward operative in vivo fluorescence imaging of the c-Met proto-oncogene for personalization of therapy in ovarian cancer. Cancer 121, 202-13 (2015).
32. Nakamura, M. et al. Hepatocyte growth factor secreted by ovarian cancer cells stimulates peritoneal implantation via the mesothelial-mesenchymal transition of the peritoneum. Gynecol Oncol 139, 345-54 (2015).
33. Matte, I. et al. Ovarian cancer ascites enhance the migration of patient-derived peritoneal mesothelial cells via cMet pathway through HGF-dependent and -independent mechanisms. Int J Cancer 137, 289-98 (2015).
34. Kwon, Y., Smith, B.D., Zhou, Y., Kaufman, M.D. & Godwin, A.K. Effective inhibition of c-MET-mediated signaling, growth and migration of ovarian cancer cells is influenced by the ovarian tissue microenvironment. Oncogene 34, 144-53 (2015).
35. Michieli, P. et al. Mutant Met-mediated transformation is ligand-dependent and can be inhibited by HGF antagonists. Oncogene 18, 5221-31 (1999).
36. Moran-Jones, K. The Therapeutic Potential of Targeting the HGF/cMET Axis in Ovarian Cancer. Mol Diagn Ther 20, 199-212 (2016).
37. Sowter, H.M., Corps, A.N. & Smith, S.K. Hepatocyte growth factor (HGF) in ovarian epithelial tumour fluids stimulates the migration of ovarian carcinoma cells. Int J Cancer 83, 476-80 (1999).
38. Blumenschein, G.R., Jr., Mills, G.B. & Gonzalez-Angulo, A.M. Targeting the hepatocyte growth factor-cMET axis in cancer therapy. J Clin Oncol 30, 3287-96 (2012).
39. Barreiros, A.P. et al. EGF and HGF levels are increased during active HBV infection and enhance survival signaling through extracellular matrix interactions in primary human hepatocytes. Int J Cancer 124, 120-9 (2009).
40. Okamoto, T. et al. Increased hepatocyte growth factor in serum in acute graft-versus-host disease. Bone Marrow Transplant 28, 197-200 (2001).
41. Yamada, T. et al. Serum interleukin-6, interleukin-8, hepatocyte growth factor, and nitric oxide changes during thoracic surgery. World J Surg 22, 783-90 (1998).
42. Jin, H. et al. Early treatment with hepatocyte growth factor improves cardiac function in experimental heart failure induced by myocardial infarction. J Pharmacol Exp Ther 304, 654-60 (2003).
43. Huh, C.G. et al. Hepatocyte growth factor/c-met signaling pathway is required for efficient liver regeneration and repair. Proc Natl Acad Sci U S A 101, 4477-82 (2004).
44. Matsumoto, K. & Nakamura, T. Hepatocyte growth factor: renotropic role and potential therapeutics for renal diseases. Kidney Int 59, 2023-38 (2001).
45. Kim, H.J. et al. Humanized Anti-hepatocyte Growth Factor Monoclonal Antibody (YYB-101) Inhibits Ovarian Cancer Progression. Front Oncol 9, 571 (2019).

## Claims

1. A method for prognosing disease progression in a patient that has been diagnosed with ovarian cancer, wherein the method comprises determining a level of hepatocyte growth factor (HGF) in a sample from the patient, wherein said level of HGF in said sample is indicative of a subsequent adverse event in the health of said patient.

2. The method according to claim 1, comprising additionally risk stratification, therapy guidance, therapy stratification and/or therapy monitoring of the patient.

3. The method according to any one of the preceding claims, wherein the subsequent adverse event in the health of said patient is cancer progression after therapeutic intervention, cancer recurrence after surgical removal and/or chemotherapy, such as cancer recurrence within 6 months after completion of chemotherapy, detection and/or development of chemotherapy resistance, and/or death.

4. The method according to the preceding claim, wherein therapeutic intervention and/or chemotherapy include administration of platin- and/or taxan-based chemotherapy agents.

5. The method according to any one of the preceding claims, wherein said sample is a blood sample, such as a serum, plasma or whole blood sample.

6. The method according to any one of the preceding claims, wherein the sample has been isolated upon initial diagnosis of said ovarian cancer and before initiation of therapeutic measures.

7. The method according to any one of the preceding claims, wherein the sample has been isolated after therapeutic intervention, for example after surgical removal of affected tissues.

8. The method according to any one of the preceding claims, wherein the sample has been isolated before, during or after therapeutic intervention, such as before, during or after administration of chemotherapy.

9. The method according to any one of the preceding claims, wherein the method comprises determining a level of hepatocyte growth factor (HGF) in a sample from the patient at more than one time points at and/or after diagnosis of said ovarian cancer.

10. The method according to any one of the preceding claims, wherein the method comprises:
- providing a sample from said patient, and
- determining a level of HGF in said sample,
- wherein said level of HGF indicates a likelihood of an adverse event in the health of said patient.

11. Method according to claim 10, wherein a determined level of HGF equal to or below a threshold value (low severity level) is indicative of the absence of a subsequent adverse event in the health of said patient, and a determined level of HGF above said threshold value (high severity level) is indicative of the occurrence of a subsequent adverse event in the health of said patient.

12. The method according to claim 11, wherein
- the low severity level of HGF indicates initiation or continuation of a routine treatment of said ovarian cancer, or
- the high severity level of HGF indicates modifying a routine treatment of said ovarian cancer.

13. The method according to the preceding claim, wherein modifying the treatment involves a change in one or more of the chemotherapeutic agents, inclusion of additional therapeutic agents in the treatment (and maintenance treatment), such as PARP inhibitors, Bevacizumab and/or modulators of HGF signaling.

14. Kit for carrying out the method for prognosing disease progression in a patient that has been diagnosed with ovarian cancer according to any one of the preceding claims, comprising:
- detection reagents for determining the level of HGF in a sample from a patient, and
- reference data, or means to obtain reference data, for the risk of a patient as to whether a subsequent adverse event in the health of said patient will occur, wherein preferably said reference data is stored on a computer readable medium and/or employed in the form of computer executable code configured for comparing the determined levels of HGF with the reference data, such as the HGF severity levels, risk threshold or cut-off value.


SEQUENCE LISTING

<110> Technische Universität Dresden

<120> HGF AS A MARKER FOR PRGRESSION OF OVARIAN CANCER

<130> 2156/20EP

<160> 6

<170> BiSSAP 1.3.6

<210> 1
<211> 728
<212> PRT
<213> Homo sapiens


<220>
<223> Amino acid sequence HGF Isoform 1 (UniProt identifier: P14210-1)

<400> 1
Met Trp Val Thr Lys Leu Leu Pro Ala Leu Leu Leu Gln His Val Leu
1               5                   10                  15
Leu His Leu Leu Leu Leu Pro Ile Ala Ile Pro Tyr Ala Glu Gly Gln
            20                  25                  30
Arg Lys Arg Arg Asn Thr Ile His Glu Phe Lys Lys Ser Ala Lys Thr
        35                  40                  45
Thr Leu Ile Lys Ile Asp Pro Ala Leu Lys Ile Lys Thr Lys Lys Val
    50                  55                  60
Asn Thr Ala Asp Gln Cys Ala Asn Arg Cys Thr Arg Asn Lys Gly Leu
65                  70                  75                  80
Pro Phe Thr Cys Lys Ala Phe Val Phe Asp Lys Ala Arg Lys Gln Cys
                85                  90                  95
Leu Trp Phe Pro Phe Asn Ser Met Ser Ser Gly Val Lys Lys Glu Phe
            100                 105                 110
Gly His Glu Phe Asp Leu Tyr Glu Asn Lys Asp Tyr Ile Arg Asn Cys
        115                 120                 125
Ile Ile Gly Lys Gly Arg Ser Tyr Lys Gly Thr Val Ser Ile Thr Lys
    130                 135                 140
Ser Gly Ile Lys Cys Gln Pro Trp Ser Ser Met Ile Pro His Glu His
145                 150                 155                 160
Ser Phe Leu Pro Ser Ser Tyr Arg Gly Lys Asp Leu Gln Glu Asn Tyr
                165                 170                 175
Cys Arg Asn Pro Arg Gly Glu Glu Gly Gly Pro Trp Cys Phe Thr Ser
            180                 185                 190
Asn Pro Glu Val Arg Tyr Glu Val Cys Asp Ile Pro Gln Cys Ser Glu
        195                 200                 205
Val Glu Cys Met Thr Cys Asn Gly Glu Ser Tyr Arg Gly Leu Met Asp
    210                 215                 220
His Thr Glu Ser Gly Lys Ile Cys Gln Arg Trp Asp His Gln Thr Pro
225                 230                 235                 240
His Arg His Lys Phe Leu Pro Glu Arg Tyr Pro Asp Lys Gly Phe Asp
                245                 250                 255
Asp Asn Tyr Cys Arg Asn Pro Asp Gly Gln Pro Arg Pro Trp Cys Tyr
            260                 265                 270
Thr Leu Asp Pro His Thr Arg Trp Glu Tyr Cys Ala Ile Lys Thr Cys
        275                 280                 285
Ala Asp Asn Thr Met Asn Asp Thr Asp Val Pro Leu Glu Thr Thr Glu
    290                 295                 300
Cys Ile Gln Gly Gln Gly Glu Gly Tyr Arg Gly Thr Val Asn Thr Ile
305                 310                 315                 320
Trp Asn Gly Ile Pro Cys Gln Arg Trp Asp Ser Gln Tyr Pro His Glu

325                 330                 335
His Asp Met Thr Pro Glu Asn Phe Lys Cys Lys Asp Leu Arg Glu Asn
            340                 345                 350
Tyr Cys Arg Asn Pro Asp Gly Ser Glu Ser Pro Trp Cys Phe Thr Thr
        355                 360                 365
Asp Pro Asn Ile Arg Val Gly Tyr Cys Ser Gln Ile Pro Asn Cys Asp
    370                 375                 380
Met Ser His Gly Gln Asp Cys Tyr Arg Gly Asn Gly Lys Asn Tyr Met
385                 390                 395                 400
Gly Asn Leu Ser Gln Thr Arg Ser Gly Leu Thr Cys Ser Met Trp Asp
                405                 410                 415
Lys Asn Met Glu Asp Leu His Arg His Ile Phe Trp Glu Pro Asp Ala
            420                 425                 430
Ser Lys Leu Asn Glu Asn Tyr Cys Arg Asn Pro Asp Asp Asp Ala His
        435                 440                 445
Gly Pro Trp Cys Tyr Thr Gly Asn Pro Leu Ile Pro Trp Asp Tyr Cys
    450                 455                 460
Pro Ile Ser Arg Cys Glu Gly Asp Thr Thr Pro Thr Ile Val Asn Leu
465                 470                 475                 480
Asp His Pro Val Ile Ser Cys Ala Lys Thr Lys Gln Leu Arg Val Val
                485                 490                 495
Asn Gly Ile Pro Thr Arg Thr Asn Ile Gly Trp Met Val Ser Leu Arg
            500                 505                 510
Tyr Arg Asn Lys His Ile Cys Gly Gly Ser Leu Ile Lys Glu Ser Trp
        515                 520                 525
Val Leu Thr Ala Arg Gln Cys Phe Pro Ser Arg Asp Leu Lys Asp Tyr
    530                 535                 540
Glu Ala Trp Leu Gly Ile His Asp Val His Gly Arg Gly Asp Glu Lys
545                 550                 555                 560
Cys Lys Gln Val Leu Asn Val Ser Gln Leu Val Tyr Gly Pro Glu Gly
                565                 570                 575
Ser Asp Leu Val Leu Met Lys Leu Ala Arg Pro Ala Val Leu Asp Asp
            580                 585                 590
Phe Val Ser Thr Ile Asp Leu Pro Asn Tyr Gly Cys Thr Ile Pro Glu
        595                 600                 605
Lys Thr Ser Cys Ser Val Tyr Gly Trp Gly Tyr Thr Gly Leu Ile Asn
    610                 615                 620
Tyr Asp Gly Leu Leu Arg Val Ala His Leu Tyr Ile Met Gly Asn Glu
625                 630                 635                 640
Lys Cys Ser Gln His His Arg Gly Lys Val Thr Leu Asn Glu Ser Glu
                645                 650                 655
Ile Cys Ala Gly Ala Glu Lys Ile Gly Ser Gly Pro Cys Glu Gly Asp
            660                 665                 670
Tyr Gly Gly Pro Leu Val Cys Glu Gln His Lys Met Arg Met Val Leu
        675                 680                 685
Gly Val Ile Val Pro Gly Arg Gly Cys Ala Ile Pro Asn Arg Pro Gly
    690                 695                 700
Ile Phe Val Arg Val Ala Tyr Tyr Ala Lys Trp Ile His Lys Ile Ile
705                 710                 715                 720
Leu Thr Tyr Lys Val Pro Gln Ser
                725

<210> 2
<211> 290
<212> PRT
<213> Homo sapiens

<220>
<223> Amino acid sequence HGF Isoform 2 (UniProt identifier: P14210-2)

<400> 2
Met Trp Val Thr Lys Leu Leu Pro Ala Leu Leu Leu Gln His Val Leu

1                   5                   10                  15
Leu His Leu Leu Leu Leu Pro Ile Ala Ile Pro Tyr Ala Glu Gly Gln
            20                  25                  30
Arg Lys Arg Arg Asn Thr Ile His Glu Phe Lys Lys Ser Ala Lys Thr
        35                  40                  45
Thr Leu Ile Lys Ile Asp Pro Ala Leu Lys Ile Lys Thr Lys Lys Val
    50                  55                  60
Asn Thr Ala Asp Gln Cys Ala Asn Arg Cys Thr Arg Asn Lys Gly Leu
65                  70                  75                  80
Pro Phe Thr Cys Lys Ala Phe Val Phe Asp Lys Ala Arg Lys Gln Cys
                85                  90                  95
Leu Trp Phe Pro Phe Asn Ser Met Ser Ser Gly Val Lys Lys Glu Phe
            100                 105                 110
Gly His Glu Phe Asp Leu Tyr Glu Asn Lys Asp Tyr Ile Arg Asn Cys
        115                 120                 125
Ile Ile Gly Lys Gly Arg Ser Tyr Lys Gly Thr Val Ser Ile Thr Lys
    130                 135                 140
Ser Gly Ile Lys Cys Gln Pro Trp Ser Ser Met Ile Pro His Glu His
145                 150                 155                 160
Ser Phe Leu Pro Ser Ser Tyr Arg Gly Lys Asp Leu Gln Glu Asn Tyr
                165                 170                 175
Cys Arg Asn Pro Arg Gly Glu Glu Gly Gly Pro Trp Cys Phe Thr Ser
            180                 185                 190
Asn Pro Glu Val Arg Tyr Glu Val Cys Asp Ile Pro Gln Cys Ser Glu
        195                 200                 205
Val Glu Cys Met Thr Cys Asn Gly Glu Ser Tyr Arg Gly Leu Met Asp
    210                 215                 220
His Thr Glu Ser Gly Lys Ile Cys Gln Arg Trp Asp His Gln Thr Pro
225                 230                 235                 240
His Arg His Lys Phe Leu Pro Glu Arg Tyr Pro Asp Lys Gly Phe Asp
                245                 250                 255
Asp Asn Tyr Cys Arg Asn Pro Asp Gly Gln Pro Arg Pro Trp Cys Tyr
            260                 265                 270
Thr Leu Asp Pro His Thr Arg Trp Glu Tyr Cys Ala Ile Lys Thr Cys
        275                 280                 285
Glu Thr
    290

<210> 3
<211> 723
<212> PRT
<213> Homo sapiens


<220>
<223> Amino acid sequence HGF Isoform 3 (UniProt identifier: P14210-3)

<400> 3
Met Trp Val Thr Lys Leu Leu Pro Ala Leu Leu Leu Gln His Val Leu
1                   5                   10                  15
Leu His Leu Leu Leu Leu Pro Ile Ala Ile Pro Tyr Ala Glu Gly Gln
            20                  25                  30
Arg Lys Arg Arg Asn Thr Ile His Glu Phe Lys Lys Ser Ala Lys Thr
        35                  40                  45
Thr Leu Ile Lys Ile Asp Pro Ala Leu Lys Ile Lys Thr Lys Lys Val
    50                  55                  60
Asn Thr Ala Asp Gln Cys Ala Asn Arg Cys Thr Arg Asn Lys Gly Leu
65                  70                  75                  80
Pro Phe Thr Cys Lys Ala Phe Val Phe Asp Lys Ala Arg Lys Gln Cys
                85                  90                  95
Leu Trp Phe Pro Phe Asn Ser Met Ser Ser Gly Val Lys Lys Glu Phe
            100                 105                 110
Gly His Glu Phe Asp Leu Tyr Glu Asn Lys Asp Tyr Ile Arg Asn Cys

        115                 120                 125
Ile Ile Gly Lys Gly Arg Ser Tyr Lys Gly Thr Val Ser Ile Thr Lys
    130                 135                 140
Ser Gly Ile Lys Cys Gln Pro Trp Ser Ser Met Ile Pro His Glu His
145                 150                 155                 160
Ser Tyr Arg Gly Lys Asp Leu Gln Glu Asn Tyr Cys Arg Asn Pro Arg
                165                 170                 175
Gly Glu Glu Gly Gly Pro Trp Cys Phe Thr Ser Asn Pro Glu Val Arg
            180                 185                 190
Tyr Glu Val Cys Asp Ile Pro Gln Cys Ser Glu Val Glu Cys Met Thr
        195                 200                 205
Cys Asn Gly Glu Ser Tyr Arg Gly Leu Met Asp His Thr Glu Ser Gly
    210                 215                 220
Lys Ile Cys Gln Arg Trp Asp His Gln Thr Pro His Arg His Lys Phe
225                 230                 235                 240
Leu Pro Glu Arg Tyr Pro Asp Lys Gly Phe Asp Asp Asn Tyr Cys Arg
                245                 250                 255
Asn Pro Asp Gly Gln Pro Arg Pro Trp Cys Tyr Thr Leu Asp Pro His
            260                 265                 270
Thr Arg Trp Glu Tyr Cys Ala Ile Lys Thr Cys Ala Asp Asn Thr Met
        275                 280                 285
Asn Asp Thr Asp Val Pro Leu Glu Thr Thr Glu Cys Ile Gln Gly Gln
    290                 295                 300
Gly Glu Gly Tyr Arg Gly Thr Val Asn Thr Ile Trp Asn Gly Ile Pro
305                 310                 315                 320
Cys Gln Arg Trp Asp Ser Gln Tyr Pro His Glu His Asp Met Thr Pro
                325                 330                 335
Glu Asn Phe Lys Cys Lys Asp Leu Arg Glu Asn Tyr Cys Arg Asn Pro
            340                 345                 350
Asp Gly Ser Glu Ser Pro Trp Cys Phe Thr Thr Asp Pro Asn Ile Arg
        355                 360                 365
Val Gly Tyr Cys Ser Gln Ile Pro Asn Cys Asp Met Ser His Gly Gln
    370                 375                 380
Asp Cys Tyr Arg Gly Asn Gly Lys Asn Tyr Met Gly Asn Leu Ser Gln
385                 390                 395                 400
Thr Arg Ser Gly Leu Thr Cys Ser Met Trp Asp Lys Asn Met Glu Asp
                405                 410                 415
Leu His Arg His Ile Phe Trp Glu Pro Asp Ala Ser Lys Leu Asn Glu
            420                 425                 430
Asn Tyr Cys Arg Asn Pro Asp Asp Asp Ala His Gly Pro Trp Cys Tyr
        435                 440                 445
Thr Gly Asn Pro Leu Ile Pro Trp Asp Tyr Cys Pro Ile Ser Arg Cys
    450                 455                 460
Glu Gly Asp Thr Thr Pro Thr Ile Val Asn Leu Asp His Pro Val Ile
465                 470                 475                 480
Ser Cys Ala Lys Thr Lys Gln Leu Arg Val Val Asn Gly Ile Pro Thr
                485                 490                 495
Arg Thr Asn Ile Gly Trp Met Val Ser Leu Arg Tyr Arg Asn Lys His
            500                 505                 510
Ile Cys Gly Gly Ser Leu Ile Lys Glu Ser Trp Val Leu Thr Ala Arg
        515                 520                 525
Gln Cys Phe Pro Ser Arg Asp Leu Lys Asp Tyr Glu Ala Trp Leu Gly
    530                 535                 540
Ile His Asp Val His Gly Arg Gly Asp Glu Lys Cys Lys Gln Val Leu
545                 550                 555                 560
Asn Val Ser Gln Leu Val Tyr Gly Pro Glu Gly Ser Asp Leu Val Leu
                565                 570                 575
Met Lys Leu Ala Arg Pro Ala Val Leu Asp Asp Phe Val Ser Thr Ile
            580                 585                 590
Asp Leu Pro Asn Tyr Gly Cys Thr Ile Pro Glu Lys Thr Ser Cys Ser
        595                 600                 605
Val Tyr Gly Trp Gly Tyr Thr Gly Leu Ile Asn Tyr Asp Gly Leu Leu
    610                 615                 620

Arg Val Ala His Leu Tyr Ile Met Gly Asn Glu Lys Cys Ser Gln His
625                 630                 635                 640
His Arg Gly Lys Val Thr Leu Asn Glu Ser Glu Ile Cys Ala Gly Ala
                645                 650                 655
Glu Lys Ile Gly Ser Gly Pro Cys Glu Gly Asp Tyr Gly Gly Pro Leu
            660                 665                 670
Val Cys Glu Gln His Lys Met Arg Met Val Leu Gly Val Ile Val Pro
        675                 680                 685
Gly Arg Gly Cys Ala Ile Pro Asn Arg Pro Gly Ile Phe Val Arg Val
    690                 695                 700
Ala Tyr Tyr Ala Lys Trp Ile His Lys Ile Ile Leu Thr Tyr Lys Val
705                 710                 715                 720
Pro Gln Ser


<210> 4
<211> 296
<212> PRT
<213> Homo sapiens


<220>
<223> Amino acid sequence HGF Isoform 4 (UniProt identifier: P14210-4)

<400> 4
Met Trp Val Thr Lys Leu Leu Pro Ala Leu Leu Leu Gln His Val Leu
1               5                   10                  15
Leu His Leu Leu Leu Leu Pro Ile Ala Ile Pro Tyr Ala Glu Gly Gln
            20                  25                  30
Arg Lys Arg Arg Asn Thr Ile His Glu Phe Lys Lys Ser Ala Lys Thr
        35                  40                  45
Thr Leu Ile Lys Ile Asp Pro Ala Leu Lys Ile Lys Thr Lys Lys Val
    50                  55                  60
Asn Thr Ala Asp Gln Cys Ala Asn Arg Cys Thr Arg Asn Lys Gly Leu
65                  70                  75                  80
Pro Phe Thr Cys Lys Ala Phe Val Phe Asp Lys Ala Arg Lys Gln Cys
                85                  90                  95
Leu Trp Phe Pro Phe Asn Ser Met Ser Ser Gly Val Lys Lys Glu Phe
            100                 105                 110
Gly His Glu Phe Asp Leu Tyr Glu Asn Lys Asp Tyr Ile Arg Asn Cys
        115                 120                 125
Ile Ile Gly Lys Gly Arg Ser Tyr Lys Gly Thr Val Ser Ile Thr Lys
    130                 135                 140
Ser Gly Ile Lys Cys Gln Pro Trp Ser Ser Met Ile Pro His Glu His
145                 150                 155                 160
Ser Phe Leu Pro Ser Ser Tyr Arg Gly Lys Asp Leu Gln Glu Asn Tyr
                165                 170                 175
Cys Arg Asn Pro Arg Gly Glu Glu Gly Gly Pro Trp Cys Phe Thr Ser
            180                 185                 190
Asn Pro Glu Val Arg Tyr Glu Val Cys Asp Ile Pro Gln Cys Ser Glu
        195                 200                 205
Val Glu Cys Met Thr Cys Asn Gly Glu Ser Tyr Arg Gly Leu Met Asp
    210                 215                 220
His Thr Glu Ser Gly Lys Ile Cys Gln Arg Trp Asp His Gln Thr Pro
225                 230                 235                 240
His Arg His Lys Phe Leu Pro Glu Arg Tyr Pro Asp Lys Gly Phe Asp
                245                 250                 255
Asp Asn Tyr Cys Arg Asn Pro Asp Gly Gln Pro Arg Pro Trp Cys Tyr
            260                 265                 270
Thr Leu Asp Pro His Thr Arg Trp Glu Tyr Cys Ala Ile Lys Asn Met
        275                 280                 285
Arg Asp Ile Thr Trp Ala Leu Asn
    290                 295

<210> 5
<211> 285
<212> PRT
<213> Homo sapiens

<220>
<223> Amino acid sequence HGF Isoform 5 (UniProt identifier: P14210-5)

<400> 5
Met Trp Val Thr Lys Leu Leu Pro Ala Leu Leu Leu Gln His Val Leu
1               5                   10                  15
Leu His Leu Leu Leu Leu Pro Ile Ala Ile Pro Tyr Ala Glu Gly Gln
            20                  25                  30
Arg Lys Arg Arg Asn Thr Ile His Glu Phe Lys Lys Ser Ala Lys Thr
        35                  40                  45
Thr Leu Ile Lys Ile Asp Pro Ala Leu Lys Ile Lys Thr Lys Lys Val
    50                  55                  60
Asn Thr Ala Asp Gln Cys Ala Asn Arg Cys Thr Arg Asn Lys Gly Leu
65                  70                  75                  80
Pro Phe Thr Cys Lys Ala Phe Val Phe Asp Lys Ala Arg Lys Gln Cys
                85                  90                  95
Leu Trp Phe Pro Phe Asn Ser Met Ser Ser Gly Val Lys Lys Glu Phe
            100                 105                 110
Gly His Glu Phe Asp Leu Tyr Glu Asn Lys Asp Tyr Ile Arg Asn Cys
        115                 120                 125
Ile Ile Gly Lys Gly Arg Ser Tyr Lys Gly Thr Val Ser Ile Thr Lys
    130                 135                 140
Ser Gly Ile Lys Cys Gln Pro Trp Ser Ser Met Ile Pro His Glu His
145                 150                 155                 160
Ser Tyr Arg Gly Lys Asp Leu Gln Glu Asn Tyr Cys Arg Asn Pro Arg
                165                 170                 175
Gly Glu Glu Gly Gly Pro Trp Cys Phe Thr Ser Asn Pro Glu Val Arg
            180                 185                 190
Tyr Glu Val Cys Asp Ile Pro Gln Cys Ser Glu Val Glu Cys Met Thr
        195                 200                 205
Cys Asn Gly Glu Ser Tyr Arg Gly Leu Met Asp His Thr Glu Ser Gly
    210                 215                 220
Lys Ile Cys Gln Arg Trp Asp His Gln Thr Pro His Arg His Lys Phe
225                 230                 235                 240
Leu Pro Glu Arg Tyr Pro Asp Lys Gly Phe Asp Asp Asn Tyr Cys Arg
                245                 250                 255
Asn Pro Asp Gly Gln Pro Arg Pro Trp Cys Tyr Thr Leu Asp Pro His
            260                 265                 270
Thr Arg Trp Glu Tyr Cys Ala Ile Lys Thr Cys Glu Thr
        275                 280                 285

<210> 6
<211> 210
<212> PRT
<213> Homo sapiens

<220>
<223> Amino acid sequence HGF Isoform 6 (UniProt identifier: P14210-6)

<400> 6
Met Trp Val Thr Lys Leu Leu Pro Ala Leu Leu Leu Gln His Val Leu
1               5                   10                  15
Leu His Leu Leu Leu Leu Pro Ile Ala Ile Pro Tyr Ala Glu Gly Gln
            20                  25                  30
Arg Lys Arg Arg Asn Thr Ile His Glu Phe Lys Lys Ser Ala Lys Thr

35                      40                      45
Thr Leu Ile Lys Ile Asp Pro Ala Leu Lys Ile Lys Thr Lys Lys Val
    50                      55                      60
Asn Thr Ala Asp Gln Cys Ala Asn Arg Cys Thr Arg Asn Lys Gly Leu
65                      70                      75                      80
Pro Phe Thr Cys Lys Ala Phe Val Phe Asp Lys Ala Arg Lys Gln Cys
                85                      90                      95
Leu Trp Phe Pro Phe Asn Ser Met Ser Ser Gly Val Lys Lys Glu Phe
            100                     105                     110
Gly His Glu Phe Asp Leu Tyr Glu Asn Lys Asp Tyr Ile Arg Asn Cys
        115                     120                     125
Ile Ile Gly Lys Gly Arg Ser Tyr Lys Gly Thr Val Ser Ile Thr Lys
    130                     135                     140
Ser Gly Ile Lys Cys Gln Pro Trp Ser Ser Met Ile Pro His Glu His
145                     150                     155                     160
Ser Phe Leu Pro Ser Ser Tyr Arg Gly Lys Asp Leu Gln Glu Asn Tyr
                165                     170                     175
Cys Arg Asn Pro Arg Gly Glu Glu Gly Gly Pro Trp Cys Phe Thr Ser
            180                     185                     190
Asn Pro Glu Val Arg Tyr Glu Val Cys Asp Ile Pro Gln Cys Ser Glu
        195                     200                     205
Gly Lys
    210